# EUROPEAN PATENT SPECIFICATION

(11) **EP 3 701 966 B1**
(45) Date of publication and mention of the grant of the patent: **09.07.2025**
(21) Application number: 18871510.6
(22) Date of filing: 26.10.2018
(51) Int. Cl.: A61K 38/16, A61K 39/29, A61P 31/20

(54) **HEPALATIDE-DERIVED COMPOUNDS FOR USE IN THE TREATMENT OF HBV-CONDITIONS**
HEPALATIDE VERBINDUNGEN ZUR VERWENDUNG BEI DER BEHANDLUNG VON HBV-KRANKHEITEN
COMPOSÉS DÉRIVÉS DE L'HÉPATIDE POUR UTILISATION DANS LE TRAITEMENT DES AFFECTIONS LIÉES AU VHB

(30) Priority: 27.10.2017 CN 201711024391
(43) Date of publication of application: 02.09.2020
(73) Proprietor: Shanghai Hep Pharmaceutical Co., Ltd., Shanghai 201203 (CN)
(72) Inventor: LIU, Hongli, Shanghai 201203 (CN)
(74) Representative: V.O.
(86) International application number: PCT/CN2018/112062
(87) International publication number: WO 2019/080919

(56) References cited:
- EP-A1- 3 025 723
- WO-A1-2009/092612
- WO-A1-2015/000371
- WO-A2-2016/055534
- CN-A- 1 733 798
- CN-A- 102 241 744
- CN-A- 106 046 155
- BLANK ANTJE ET AL: "First-in-human application of the novel hepatitis B and hepatitis D virus entry inhibitor myrcludex B", JOURNAL OF HEPATOLOGY, ELSEVIER, AMSTERDAM, NL, vol. 65, no. 3, 27 April 2016 (2016-04-27), pages 483 - 489, XP029703163, ISSN: 0168-8278, DOI: 10.1016/J.JHEP.2016.04.013
- GRIPON, P.: "Efficient Inhibition of Hepatitis B Virus Infection by Acyla- ted Peptides Derived from the Large Viral Surface Protein", JOURNAL OF VIROLOGY, vol. 79, no. 3, 28 February 2005 (2005-02-28), pages 1613 - 1622, XP002494027
- PETERSEN, J.: "Prevention of hepatitis B virus infection in vivo by entry inhibitors derived from the large envelope protein", NATURE BIOTECHNOLOGY, vol. 26, no. 3, 24 February 2008 (2008-02-24), pages 335 - 341, XP002494028, DOI: 10.1038/nbt1389
- KURODA, S.: "Hepatitis B Virus Envelope L Protein Particles SYNTHESIS AND ASSEMBLY IN SACCHAROMYCES CEREVISIAE, PURIFICATION AND CHARACTERIZATION", THE JOURNAL OF BIOLOGICAL CHEMISTRY, vol. 267, no. 3, 25 January 1992 (1992-01-25), pages 1953 - 1961, XP002937464
- DENG, TAO ET AL.: "EXPRESSION OF Fas LIGAND ON HBVCTLs AND THE DYNAMIC LEVEL OF NITRIC OXIDE IN SUPERNATANT OF HBVCTLs INDUCED BY SYNTHETIC PEPTIDES OF HBcAg", CHINESE JOURNAL OF INMUNOLOGY, vol. 14, no. 2, 31 December 1998 (1998-12-31), pages 144 - 146, XP009520476, ISSN: 1000-484X

## Description

### Technical Field

This invention relates to a polypeptide comprising an amino acid sequence selected from SEQ ID NOs: 21 - 40 and 49 or a pharmaceutical composition comprising said polypeptide for use in methods for treating or preventing hepatitis B virus-related liver diseases in a dose of 4.2 - 5.0mg for at least 7 consecutive days.

### Background

About one million people die every year in the world from liver failure, cirrhosis and primary hepatocellular carcinoma caused by hepatitis B virus (HBV) infection. At present, it is estimated that in our country there are 20 million existing chronic hepatitis B patients, 1 million new cases every year, 5 million cirrhosis cases caused by hepatitis B every year, and 1 million deaths every year. Every year, 300000 cases of hepatitis B-related liver cancer occur and 300000 cases die.

HBV viral envelope contains three surface antigen proteins: large (L), medium (M), and small (S). These proteins are coded by a single open reading frame on the S gene, starting from three different translation initiation sites, i.e., L (Pre-S1 + Pre-S2 + S), M (Pre-S2 + S), and S (S). HBV is divided into 9 genotypes (A-I), and Pre-S1 region of each genotype has different amino acid sequence. Research has confirmed that HBV binds to the sodium taurocholate cotransporting polypeptide (NTCP) on the surface of liver cell through the large protein (large HBsAg) on the surface of virus, and mediates the infection of liver cell (Yan et al., ELife , 1: e00049 (2012)). Derivative polypeptides containing the HBV Pre-S1 amino acid sequence can block HBV infection of hepatocytes *in vitro* (Gripon P et al. J. Virol. 2005; 79 (3): 1613-1622) and in animal models (Petersen J et al. Nat Biotechnol. 2008 Mar; 26 (3): 335-41.). In addition, NTCP bears the physiological function of transporting bile acids from the portal vein blood stream to the liver, and is one of the important proteins of bile acid enterohepatic circulation (Alrefai W et al. Pharmaceutical Research, 2007; 24 (10): 1803-1823).

NTCP is specifically expressed in the liver, and is rarely expressed in other tissues. HBV Pre-S1-derived polypeptides show obvious liver aggregation in animals (Schieck a et al. Hepatology. 2013 Jul; 58 (1): 43-53.). Phase Ia clinical studies of human single-dose climb and pharmacokinetic studies for the peptide Myrcludex B, derived from HBV Pre-S1, has completed (lank A et al. J Hepatol. 2016 Sep; 65 (3): 483-9.). In lank A's study, 12 dose groups were set up and given single 2h continuous intravenous injections at the dosages of 0.3ug, 3ug, 10ug, 100ug, 800ug, 3mg, 5mg, 10mg, and 20mg and single subcutaneous injections at the dosages of 800ug, 5mg, and 10mg, respectively. A total of 36 healthy subjects were enrolled and assigned to the above 12 dose groups, with 3 persons for each group. All 36 subjects completed clinical trials. Due to the influence of continuous intravenous administration on pharmacokinetic parameters, it is difficult to analyze the pharmacokinetic parameters of intravenous administration. Analysis of the apparent distribution volume V of subcutaneous administration showed that after single subcutaneous administrations of 800ug and 5mg, the apparent distribution volumes were both greater than 100L, which were much larger than the body weight, indicating that the peptide was distributed in the target organ; when the dosage was increased to 10mg, the blood concentration of the peptide is high, and the apparent distribution volume is 43L. Due to lank A's study used fewer dosage groups for subcutaneous administration and lacked reference and comparison, no conclusion can be drawn if the polypeptide content in the liver target organ is saturated. EP 3 025 723 A1 describes a composition comprising a polypeptide and a buffer salt, in particular a phosphate buffer salt. WO 2016/055534 A2 disclose a combination therapy of HBV and HDV infection using a composition comprising an inhibitor of Na+-taurocholate cotransporting polypeptide (NTCP) and an active agent. WO 2009/092612 A1 discloses hydrophobic modified preS-derived peptide of HBV as a vehicle for the specific delivery of compounds to the liver for use in preventing and/or treating liver diseases or disorders. Blank A et al. describe Myrcludex B for use in treating HBV and HDV wherein up to 20 mg Myrcludex B intravenously and up to 10 mg Myrcludex B subcutaneously are administered in a single dose (Journal of Hepatology, 28 April 2016). In the clinical pharmacokinetic study of Myrcludex B on continuous subcutaneous administration for 6 days at a dose of 10mg, after the administration, the accumulation coefficient R_{AUC} was 1.79, indicating an accumulation effect; however, the apparent distribution volume was not reported (Blank A et al. Clin Pharmacol Ther. 2017 May 24. doi: 10.1002/cpt.744.). The clinical trial on treating hepatitis D patients with interferon and Myrcludex B at a dose of 2mg Myrcludex B per day is in progress, wherein the dosage selection was based on the results of the pharmacokinetic study of the peptide in chimpanzees, but the clinical study results did not show the therapeutic effect on the primary end-point indicators of HBsAg clearance (Bogomolov P et al. J Hepatol. 2016 Sep; 65(3):490-8. doi: 10.1016/j.jhep.2016.04.016. Epub 2016 Apr 27). A sufficient dosage of HBV Pre-S1-derived peptide for treating HBV-related liver disease has not been determined.

### Summary

The present invention is defined by the appended claims. The technical disclosure set out below may in some respects go beyond the scope of the claims. Elements of the disclosure which do not fall within the scope of the claims are provided for information.
This disclosure provides a polypeptide or a pharmaceutical composition comprising the polypeptide per day, wherein the polypeptide comprises an amino acid sequence derived from hepatitis B virus (HBV) Pre-S1 for use in a method for treating and preventing hepatitis B virus-related liver diseases ; the sufficient dosage is a daily dosage at which the polypeptide reaches a saturating level in the liver target organ after the polypeptide is administered in the sufficient dose for several days.
The sufficient dosage described herein is a daily dosage of 777.95-926.13 nmol, preferably a daily dosage of 777.95 nmol or 926.13 nmol.
The sufficient dosage described herein is a daily dosage of 4.2~5.0 mg, preferably a daily dosage of 4.2 mg or 5.0 mg.
The polypeptide described herein comprises an amino acid sequence of the pre-S1 region of HBV.
The polypeptide described herein comprises an N-terminal modification with a hydrophobic group.
The hydrophobic group described herein is chosen from myristic acid, palmitic acid, stearic acid, oleic acid, linoleic acid, cholesterol, and arachidonic acid.
Preferably, the hydrophobic group described herein is myristic acid.
The polypeptide described herein comprises a C-terminal modification or does not comprise a C-terminal modification.
Preferably, the C-terminal modification described herein is amidation or isopentanediolization.

For example, the polypeptide described herein includes an amino acid sequence of the pre-S1 region of any one of HBV genotypes A, B, C, D, E, F, G, H and I.

For example, the polypeptide described herein comprises the sequence of amino acids 13-59 of the pre-S1 region of HBV genotype C or comprises a sequence from the pre-S1 region of HBV genotype A, B, D, E, F, G, H or I that corresponds to amino acids 13-59 of the pre-S1 region of HBV genotype C.

For example, one or more amino acid residues of the polypeptide described herein are deleted, substituted, or inserted; preferably, 1-30, 1-20, 1-10, 1-8, 1-5, or 1-3 amino acid residues of the polypeptide described herein are deleted, substituted, or inserted.

The polypeptide described herein comprises at the N-terminus and/or the C-terminus a native flanking amino acid sequence from the pre-S1 region of HBV; preferably, the native flanking amino acid sequence from the pre-S1 region of HBV has 1-10, 1-8, 1-5, or 1-3 amino acids in length.

For example, the polypeptide described herein comprises the glycine corresponding to amino acid 13 of the pre-S1 region of HBV genotype C, and/or the asparagine corresponding to amino acid 20 or the lysine corresponding to amino acid 57 of the pre-S1 region of HBV genotype C.

According to the invention, the polypeptide described herein:
(1) comprises an amino acid sequence selected from SEQ ID NOs: 21-40 or 49; preferably, the polypeptide comprises the amino acid sequence of SEQ ID NO: 23.

In some embodiments, the polypeptide described herein is any one of SEQ ID NOs: 1-20 or 51.

In some embodiments, the polypeptide described herein comprises SEQ ID NO: 23 or 49, wherein the polypeptide comprises an N-terminal modification with myristic acid and a C-terminal modification with amination, or wherein the polypeptide comprises SEQ ID NO: 3 or 51.

In some embodiments, the hepatitis B virus-related diseases describe herein include chronic hepatitis B virus infection, chronic hepatitis B, hepatitis B-related liver fibrosis and cirrhosis, hepatitis B-related liver cancer, hepatitis B-related liver transplantation and hepatitis B-related mother-to-child transmission.

In some embodiments, the chronic hepatitis B described herein includes HBeAg positive chronic hepatitis B and HBeAg negative chronic hepatitis B.

In some embodiments, the hepatitis B-related liver transplantation described herein includes the protection of the donor liver from HBV infection before, during and after transplantation.

In some embodiments, the polypeptide described herein comprises the amino acid sequence of SEQ ID NO: 23, and comprises an N-terminal modification with myristic acid and a C-terminal modification with amination; the sufficient dosage described herein is a daily dosage of 4.2-5.0 mg, preferably a daily dosage of 4.2 mg, the method described herein comprises continuous administration for at least 7 days.

In some embodiments, the polypeptide described herein comprises the amino acid sequence of SEQ ID NO: 49, and comprises an N-terminal modification with myristic acid and a C-terminal modification with amination; the sufficient dosage described herein is a daily dosage of 4.2-5.0 mg, preferably a daily dosage of 5.0 mg, the method described herein comprises continuous administration for at least 7 days.

In some embodiments, the polypeptide described herein comprises the amino acid sequence of SEQ ID NO: 23, and comprises an N-terminal modification with myristic acid and a C-terminal modification with amination, the method described herein comprises administering N doses of the pharmaceutical composition to the subject daily to achieve a daily dosage of 4.2 mg, wherein N is any integer from 1 to 42.

In some embodiments, the polypeptide described herein comprises the amino acid sequence of SEQ ID NO: 49, and comprises an N-terminal modification with myristic acid and a C-terminal modification with amination, the method described herein comprises administering N doses of the pharmaceutical composition to the subject daily to achieve a daily dosage of 5.0 mg, wherein N is any integer from 1 to 50.

This disclosure further provides a pharmaceutical composition, the polypeptide described herein comprises an amino acid sequence derived from hepatitis B virus (HBV) Pre-S1; the pharmaceutical composition described herein is a pharmaceutical composition comprising a certain amount of the polypeptide, by administering an integer number of preparation specifications of the pharmaceutical composition, the polypeptide reaches a saturating content in a liver target organ.

The pharmaceutical composition described herein comprises a therapeutically effective amount of the polypeptide, wherein the therapeutically effective amount is an amount that after daily administration of one or several doses of the pharmaceutical composition the daily total dosage of the polypeptide reaches 777.95-926.13 nmol.

Tthe therapeutically effective amount described herein is an amount that after daily administration of one or several doses of the pharmaceutical composition the daily total dosage of the polypeptide reaches 4.2~5.0 mg, preferably 4.2 mg or 5.0 mg.

In some embodiments, each dose of the pharmaceutical composition described herein comprises 4.2 mg, 2.1 mg, 1.4 mg, 0.7 mg, 0.6 mg, 0.3 mg, 0.2 mg or 0.1 mg of the polypeptide, i.e. the specification of the pharmaceutical composition is 4.2 mg, 2.1 mg, 1.4 mg, 0.7 mg, 0.6 mg, 0.3 mg, 0.2 mg or 0.1 mg; or each dose of the pharmaceutical composition described herein comprises 5.0 mg, 2.5 mg, 1.0 mg, 0.5 mg, 0.25 mg or 0.1 mg of the polypeptide, i.e. the specification of the pharmaceutical composition is 5.0 mg, 2.5 mg, 1.0 mg, 0.5 mg, 0.25 mg or 0.1 mg.

The polypeptide described herein is the polypeptide described in any embodiment of the present disclosure.

This disclosure further describes a kit comprising one or more doses of medicaments which containing a polypeptide as an active ingredient, and which are used for administration for one or more days, wherein the amount of the polypeptide contained in the one or more doses of the medicaments described herein is an amount that after administrating one or several doses of the medicaments daily, the daily amount of the polypeptide reaches 777.95-883.52 nmol, wherein the polypeptide described herein comprises an amino acid sequence derived from hepatitis B virus (HBV) Pre-S1.

It is described for example that the amount of the polypeptide contained in the one or more doses of the medicaments described herein is an amount that after administrating one or several doses of the medicaments daily, the daily administration amount reaches 4.2~5.0 mg, preferably 4.2 mg or 5.0 mg.

Further described is for example the use of the pharmaceutical composition described herein in the manufacture of a medicament for treating or preventing hepatitis B virus-related liver diseases.

The invention refers to the polypeptide or the pharmaceutical composition comprising the polypeptide described herein for use in the treatment and prevention of hepatitis B virus-related liver diseases.

### Description of Drawings

Fig. 1 shows that hepalatide labeled with FIFC bind to NTCP expressing HEK293 cells (NTCP-293), but do not bind to control HEK293 cells (BLANK-293). A polypeptide derived from heron HBV, labeled with FIFC, was used as a control polypeptide.
Fig. 2 shows the effect of Cmyr-47 on bile acid uptake *in vitro.* Cyclosporine A (CsA) was used as a positive control. Fig. 2A shows that TA entry into cells is specifically mediated by NTCP, and the positive control cyclosporine A exerts a significant inhibitory effect on NTCP. Fig. 2B shows that hepalatide has a bidirectional effect on NTCP bile acid transport function.
Fig. 3 shows precipitation radioactivity-time curves after subcutaneous injection of ¹²⁵I-hepalatide.
Fig. 4 shows mean blood concentration-time curves (mean + SD) in Phase Ia PK study.
Fig. 5 shows average blood concentration-time curves for first dose (mean + SD) in Phase Ib PK study .
Fig. 6 shows average blood concentration-time curves (mean + SD) before administration on days 2-7 in Phase Ib PK study.
Fig. 7 shows average blood concentration-time curves for last dose (mean + SD) in Phase Ib PK study.

### Detailed Description

Unless defined otherwise, all technical and scientific terms used herein have the same meaning as commonly understood by those of ordinary skill in the art to which the disclosure belongs. For the purposes of the present disclosure, the following terms are defined below.

The articles "a" and "an" refer to one or to more than one (i.e., to at least one) of the grammatical object of the article. For example, "an element" means one element or more than one element.

The term "or" means, and is used interchangeably with, the term "and/or," unless context clearly indicates otherwise.

To the extent that the term "contain," "include," "have," or grammatical variants of such term are used in either the disclosure or the claims, such term is inclusive in a manner similar to the term "comprising" as "comprising" is interpreted when employed as a transitional word in a claim. The term "including" or its grammatical variants mean, and are used interchangeably with, the phrase "including but not limited to."

The term "about" means a quantity, level, value, number, frequency, percentage, dimension, size, amount, weight, or length that varies by as much as 30, 25, 20, 15, 10, 9, 8, 7, 6, 5, 4, 3, 2 or 1% to a reference quantity, level, value, number, frequency, percentage, dimension, size, amount, weight or length. When the term "about" is used in conjunction with a numerical range, it modifies that range by extending the boundaries above and below the numerical values set forth. In general, the term "about" is intended to modify a numerical value above and below the stated value by a variance of ≦ 10%.

This application contains a Sequence Listing which has been submitted electronically in ASCII format.

Any combination of the technical features in the embodiments described in the disclosure can be used within the scope of the disclosure to form preferred technical solutions.

### I. Polypeptides

Certain aspects of the present disclosure provide polypeptides derived from HBV Pre-S1 for use in treating or preventing HBV-related liver diseases. Preferably, the polypeptides can block HBV infection *in vitro.* More preferably, the polypeptide can bind to NTCP *in vitro,* e.g., in solution or in a cell-free system (such as cell lysate or a reconstructed system), or in cells, such as cells cultured *in vitro* (such as cells expressing NTCP, or hepatocytes), or can bind to NTCP in a subject's cells *in vivo.* The subject may be a mammal. In some embodiments, the subject may be a human.

The terms "polypeptide," "peptide," and "protein" are used interchangeably and encompass full-length proteins and fragments, as well as variants of the full-length proteins and the fragments. Such fragments and variants of the polypeptide described herein retain at least one or more biological activities of the polypeptide. The "polypeptide," "peptide," and "protein" can include natural and/or non-natural amino acid residues. Those terms also include post-translationally modified proteins, including, e.g., glycosylated, sialylated, acetylated, and/or phosphorylated proteins. The terms also include chemically modified proteins at one or more amino acid residues, such as, e.g., at the N-terminus and/or at the C-terminus. For instance, the N-terminus of the polypeptide disclosed herein can be modified by a hydrophobic group such as, e.g., myristic acid, palmitic acid, stearic acid, oleic acid, linoleic acid, cholesterol, and arachidonic acid. In some embodiments, the C-terminus of the polypeptide disclosed herein can be modified. The C-terminus modification may be chosen from amidation (amination), isopentanediolization or no-modification.

As used herein, the term "polypeptide derived from HBV Pre-S1" or "HBV Pre-S1-derived polypeptide" refers to the origin or source of the polypeptide as being from HBV, and may include native, recombinant, synthesized, or purified polypeptides. The term "polypeptide derived from HBV" or "HBV-derived polypeptide" refers to a full-length native HBV polypeptide or fragments thereof, as well as variants of the full-length native polypeptide or its fragments. The fragment may consist of at least 3-5 amino acids, at least 5-10 amino acids, at least 10-20 amino acids, at least 20-30 amino acids, at least 30-50 amino acids, or the entire amino acids of the native sequence, or may be otherwise identifiable to one of ordinary skill in the art as having its origin in the native sequence. The polypeptide described herein may be derived from the pre-S1 region of the L protein of any HBV subtype. The polypeptide described herein may comprise the entire pre-S1 region of the L protein of any HBV subtype. The polypeptide described herein may be derived from the pre-S1 region of the L protein of any one of HBV genotypes A, B, C, D, E, F, G, H, or I. The genomic sequences of these HBV genotypes can be found in GenBank Accession Nos. KC875260 (SEQ ID NO: 41), AY220704 (SEQ ID NO: 42), AF461363 (SEQ ID NO: 43), AY796030 (SEQ ID NO: 44), AB205129 (SEQ ID NO: 45), DQ823095 (SEQ ID NO: 46), HE981176 (SEQ ID NO: 47), and AB179747 (SEQ ID NO: 48), respectively. The polypeptide described herein may be derived from the pre-S1 region of the L protein of HBV genotype C. The polypeptide derived from HBV described herein retains one or more biological activities described herein of the corresponding native HBV polypeptide.

"Variant" as used herein in connection with the polypeptide described herein, a polypeptide derived from HBV Pre-S1, or an HBV Pre-S1-derived polypeptide means a polypeptide that differs from a given polypeptide (i.e., the polypeptide described herein, the polypeptide derived from HBV Pre-S1, or the HBV Pre-S1-derived polypeptide) in amino acid sequence, but retains one or more biological activities described herein of the given polypeptide. The variant polypeptide described herein may have one or more amino acid additions (e.g., insertion), deletions, or substitutions from the given polypeptide. The variant polypeptide described herein may have 1-30, 1-20, 1-10, 1-8, 1-5, or 1-3 amino acid additions (e.g., insertion), deletions, or substitutions from the given polypeptide, including all integers in between these ranges. For example, the polypeptide sequence may contain conservative substitution of amino acids. A conservative substitution of an amino acid, i.e., replacing an amino acid with a different amino acid of similar properties (e.g., hydrophilicity and degree and distribution of charged regions), typically involves a minor change and therefore does not significantly alter the biological activity of the polypeptide. These minor changes can be identified, in part, by considering the hydropathic index of amino acids based on a consideration of the hydrophobicity and charge of the amino acid. Amino acids of similar hydropathic indexes and hydrophilicity values can be substituted and still retain protein function. Both the hydrophobicity index and the hydrophilicity value of amino acids are influenced by the particular side chain of that amino acid. Consistent with that observation, amino acid substitutions that are compatible with biological function depend on the relative similarity of the amino acids, and particularly the side chains of those amino acids, as revealed by the hydrophobicity, hydrophilicity, charge, size, and other properties.

The term "variant" also includes a polypeptide that has certain identity, such as, e.g., at least about 30%, 40%, 50%, 60%, 70%, 80%, 85%, 90%, 95%, 96%, 97%, 98%, or 99% identity to the given polypeptide. "Variant" as used herein also includes a polypeptide comprising the portion of the given polypeptide that corresponds to a native sequence of HBV proteins. "Variant" may also refer to a fusion protein or chimeric protein, comprising polypeptides derived from two or more different sources. Non-limiting examples of the fusion protein described herein may include, e.g., a fusion protein of one polypeptide derived from HBV Pre-S1 and another polypeptide derived from a non-HBV Pre-S1 protein, a fusion protein of two polypeptides derived from different HBV subtypes, and a fusion protein of two polypeptides derived from different regions of the L protein of any one of HBV subtypes, or from different sequences within the pre-S1 region of the L protein of any one of HBV subtypes.

The term "variant" also includes a polypeptide that comprises the same amino acid sequence of a given polypeptide (i.e., the polypeptide described herein, the polypeptide derived from HBV Pre-S1, or the HBV Pre-S1-derived polypeptide) and retains one or more biological activities of the given polypeptide, but chemically and/or post-translationally modified in a manner different from the given polypeptide. "Variant" can also be used to describe a polypeptide or a fragment thereof that has been differentially processed, such as by proteolysis, phosphorylation, or other post-translational modification, yet retains one or more biological activities described herein. Use of "variant" herein is intended to encompass fragments of a variant unless otherwise contradicted by context. The term "variant" also encompasses the homologous polypeptide sequences found in the different viral species, strains, or subtypes of the hepadnavirus genus. HBV is divided into four major serotypes (adr, adw, ayr, ayw) based on antigenic epitopes present on its envelope proteins, and into nine genotypes (A-I) according to overall nucleotide sequence variation of the genome. The term "variant" therefore includes homologous polypeptides found in any of these HBV subtypes. "Variant" can also include polypeptides having native flanking amino acid sequences from any of these HBV subtypes added to the N and/or C terminus.

The terms "conservative amino acid substitutions" and "conservative substitutions" are used interchangeably herein to refer to intended amino acid swaps within a group of amino acids wherein an amino acid is exchanged with a different amino acid of similar size, structure, charge, and/or polarity. Families of amino acid residues having similar side chains are known in the art, including basic side chains (e.g. , lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g. , alanine, valine, leucine, isoleucine, proline, phenylalanine, methionine, tryptophan), beta-branched side chains (e.g., threonine, valine, isoleucine) and aromatic side chains (e.g. , tyrosine, phenylalanine, tryptophan, histidine). Thus, in some embodiments, an amino acid residue in a polypeptide can be replaced with another amino acid residue from the same side chain family. In other embodiments, a string of amino acids can be replaced with a structurally similar string that differs in order and/or composition of side chain family members. In yet other embodiments, mutations can be introduced randomly along all or part of the polypeptide. Examples of conservative amino acid substitutions include, e.g., exchange of one of the aliphatic or hydrophobic amino acids Ala, Val, Leu, and Ile for one of the other amino acids in that group of four; exchange between the hydroxyl-containing residues Ser and Thr; exchange between the acidic residues Asp and Glu; exchange between the amide residues Asn and Gln; exchange between the basic residues Lys, Arg, and His; exchange between the aromatic residues Phe, Tyr, and Trp; and exchange between the small-sized amino acids Ala, Ser, Thr, Met, and Gly. Conservative substitutions, such as substituting a conserved amino acid with a similar, structurally related amino acid would not be reasonably expected to impose a substantial influence on the biological activity of the polypeptide.

The term "sequence identity" (e.g., a "sequence 50% identical to") refers to the extent that a sequence is identical on an amino acid-by-amino acid basis over a window of comparison. The polypeptide described herein may comprise an amino acid sequence at least about 30%, 40%, 50%, 60%, 70%, 80%, 85%, 90%, 95%, 96%, 97%, 98%, or 99% identical to the sequence of a given polypeptide and still retain one or more biological activities of the given polypeptide. A "percentage identity" (or "% identity") may be calculated by comparing two optimally aligned sequences over the window of comparison, determining the number of positions at which the identical amino acids occur in both sequences to yield the number of matched positions, dividing the number of matched positions by the total number of positions in the window of comparison, and multiplying the result by 100 to yield the percentage of sequence identity. Optimal alignment of sequences for aligning a comparison window may be conducted by computerized implementations of algorithms available in the art, such as, e.g., the BLAST^{®} family of programs, or by visual inspection and the best alignment (i.e., resulting in the highest percentage homology over the comparison window) generated by any of the various methods selected. For sequence comparison, one sequence acts as a reference sequence, to which test sequences are compared. When using a sequence comparison algorithm, test and reference sequences are input into a computer, subsequence coordinates may be designed, if necessary, and sequence algorithm program parameters are designated. The sequence comparison algorithm then calculates the present sequence identity for the test sequences relative to the reference sequence, based on the designated program parameters. The designation of sequence algorithm program parameters is well within the knowledge in the art. For example, the window of comparison may be designated as over the entire length of either or both comparison sequences, such as, e.g., over the entire length of the reference sequence, and gaps of up to 5% of the total number of amino acids in the reference sequence may be allowed.

Tthe polypeptide described herein may comprise an amino acid sequence of the pre-S 1 region of any HBV subtype. According to the invention, the polypeptide described herein comprises the sequence of amino acids 13-59 of the pre-S1 region of HBV genotype C: GTNLSVPNPLGFFPDHQLDPAFGANSNNPDWDFNPNKDHWPEANQVG (SEQ ID NO: 23) or GTNLSVPNPLGFFPDHQLDPAFGANSNNPDWDFNPNKDHWPEANKVG (SEQ ID NO: 49). The polypeptide described herein may comprise the corresponding pre-S1 sequence from another HBV genotype, such as, e.g., any one of genotypes A, B, D, E, F, G, H, or I. According to the invention, the polypeptide described herein may comprise:
pre-S1 amino acids 13-59 of HBV genotype A: GTNLSVPNPLGFFPDHQLDPAFGAN SNNPDWDFNPVKDDWPAANQVG (SEQ ID NO: 34),
pre-S1 amino acids 13-59 of HBV genotype B: GTNLSVPNPLGFFPDHQLDPAFKAN SENPDWDLNPNKDNWPDANKVG (SEQ ID NO: 35),
pre-S1 amino acids 2-48 of the HBV genotype D: GQNLSTSNPLGFFPDHQLDPAFRA NTANPDWDFNPNKDTWPDANKVG (SEQ ID NO: 36),
pre-S1 amino acids 12-58 of the HBV genotype E: GKNISTTNPLGFFPDHQLDPAFRA NTRNPDWDHNPNKDHWTEANKVG (SEQ ID NO: 37),
pre-S1 amino acids 13-59 of the HBV genotype F: GQNLSVPNPLGFFPDHQLDPLFRA NSSSPDWDFNTNKDSWPMANKVG (SEQ ID NO: 38),
pre-S1 amino acids 12-58 of the HBV genotype G: GKNLSASNPLGFLPDHQLDPAFR ANTNNPDWDFNPKKDPWPEANKVG (SEQ ID NO: 39), or
pre-S1 amino acids 13-59 of the HBV genotype H: GQNLSVPNPLGFFPDHQLDPLFR ANSSSPDWDFNTNKDNWPMANKVG (SEQ ID NO: 40).

According to the invention, the polypeptide described herein comprises a portion of the pre-S1 region of HBV, said portion comprising at least an amino acid sequence chosen from SEQ ID NOs: 23, 34-40 or 49. In some embodiments, the polypeptide described herein may comprise the entire pre-S1 region of HBV.
The polypeptide described herein may be 10-100 amino acids in length. For example, the polypeptide may be 15-100, 15-80, 20-100, 20-80, 20-60, 25-60, 30-60, 35-60, or 40-60 amino acids in length, including all integers in between these ranges. In some embodiments, the polypeptide described herein may be at least 20, such as, e.g., at least 25, 30, 35, 40, amino acids in length. In some embodiments, the polypeptide described herein may be 20, 25, 30, 35, 40, 47, 55, 60 amino acids in length. In some embodiments, the polypeptide described herein may be 47 amino acids in length. The variants of the polypeptides described herein that differ in length retain one or more biological activities associated with the corresponding polypeptides.

In some embodiments, the polypeptide described herein may comprise an N-terminal modification with a hydrophobic group. For example, the hydrophobic group may be chosen from myristic acid, palmitic acid, stearic acid, oleic acid, linoleic acid, cholesterol, and arachidonic acid. In some embodiments, the hydrophobic group may be chosen from myristic acid, palmitic acid, stearic acid, and cholesterol. In some embodiments, the hydrophobic group may be myristic acid. In certain embodiments, the polypeptide described herein comprises an amino acid sequence chosen from SEQ ID NOs: 23, 34-40 and 49, wherein the N terminus may be modified with a hydrophobic group chosen from myristic acid, palmitic acid, stearic acid, and cholesterol. In certain embodiments, the polypeptide described herein comprises an amino acid sequence chosen from SEQ ID NOs: 23, 34-40 and 49, wherein the N terminus may be myristoylated. In some embodiments, the polypeptide described herein comprises the amino acid sequence of SEQ ID NO: 23, wherein the N terminus may be myristoylated. In some embodiments, the polypeptide described herein may comprise a C-terminal modification or does not comprise a C-terminal modification. For example, the C-terminal modification may be chosen from amidation (amination), isopentanediolization, or no-C-terminal-modification. In some embodiments, the C-terminal modification may be amidation (amination). For example, the polypeptide described herein comprises the amino acid sequence of SEQ ID NO: 23, wherein the N terminus may be myristoylated, and/or the C terminus may be amidated (aminated). In some embodiments, the polypeptide described herein comprises the amino acid sequence of SEQ ID NO: 23. In some embodiments, the polypeptide described herein comprises an amino acid sequence chosen from SEQ ID NOs: 34-40 and 49, wherein the N terminus may be myristoylated, and/or the C terminus may be modified by amidated (aminated). In some embodiments, the polypeptide described herein comprises an amino acid sequence chosen from SEQ ID NOs: 14-20 and 51. The variants of the polypeptide described herein that are modified at the N-terminus and/or the C-terminus retain one or more biological activities of the corresponding polypeptides that are not modified in the same manner.

Variants of the polypeptides described herein are also contemplated in the present disclosure, including variants with one or more amino acid deletions, substitutions, or insertions that retain one or more biological activities of the polypeptides. The polypeptides described herein preferably retain the glycine corresponding to amino acid 13 of the pre-S1 region of HBV genotype C (i.e., the N-terminal glycine of SEQ ID NO: 23). For example the polypeptides described herein retain the asparagine corresponding to amino acid 20 or the lysine corresponding to amino acid 57 of the pre-S1 region of HBV genotype C. Tthe polypeptide described herein may have one or more naturally-occurring mutations in the pre-S1 region of HBV. The polypeptide described herein may have 1-30, such as, e.g., 1-20, 1-10, 1-8, 1-5, or 1-3, amino acid deletions, substitutions, or insertions relative to a sequence from the pre-S1 region of HBV, including all integers in between these ranges. In some embodiments, the polypeptide described herein may have 1-30, such as, e.g., 1-20, 1-10, 1-8, 1-5, or 1-3, amino acid deletions, substitutions, or insertions relative to an amino acid sequence chosen from SEQ ID NOs: 23, 34-40 and 49, including all integers in between these ranges. In some embodiments, the polypeptide described herein may have 1-30, such as, e.g., 1-20, 1-10, 1-8, 1-5, or 1-3, amino acid deletions, substitutions, or insertions relative to the amino acid sequence of SEQ ID NO: 23, including all integers in between these ranges. In some embodiments, the polypeptide described herein may have 1-3 amino acid deletions, substitutions, or insertions from the amino acid sequence of SEQ ID NO: 23. In certain embodiments, the polypeptide described herein may have 1-30, such as, e.g., 1-20, 1-10, 1-8, 1-5, or 1-3, amino acid deletions or insertions at the C terminus of an amino acid sequence chosen from SEQ ID NOs: 23, 34-40 and 49, including all integers in between these ranges. The polypeptide described herein comprises an amino acid sequence chosen from SEQ ID NOs: 21, 22, and 24-28. In some embodiments, the polypeptide described herein comprises the amino acid sequence of any one of the polypeptides listed in Table 1. In some embodiments, the polypeptide described herein may be chosen from any one of the post-translationally modified polypeptides listed in Table 1.

**Table 1. List of Exemplary Polypeptides**

| SEQ ID No. | SEQ name | N -terminal Modification | Amino acid sequence 123456789012345678901234567890 12 | C-terminal Modification | SEQ origin |
|---|---|---|---|---|---|
| 1 | Cmyr-60 | Myr | GTNLSVPNPLGFFPDHQLDPAFGANSNNPD WDFNPNKDHWPEANQVGAGAFGPGFTPPHG | NH2 | Genotype C Pre-S1(13-72) |
| 2 | Cmyr-55 | Myr | GTNLSVPNPLGFFPDHQLDPAFGANSNNPD WDFNPNKDHWPEANQVGAGAFGPGF | NH2 | Genotype C Pre-S1(13-67) |
| 3 | Cmyr-47 | Myr | GTNLSVPNPLGFFPDHQLDPAFGANSNNPD WDFNPNKDHWPEANQVG | NH2 | Genotype C Pre-S1(13-59) |
| 4 | Cmyr-40 | Myr | GTNLSVPNPLGFFPDHQLDPAFGANSNNPD WDFNPNKDHW | NH2 | Genotype C Pre-S1(13-52) |
| 5 | Cmyr-35 | Myr | GTNLSVPNPLGFFPDHQLDPAFGANSNNPD WDFNP | NH2 | Genotype C Pre-S1(13-47) |
| 6 | Cmyr-30 | Myr | GTNLSVPNPLGFFPDHQLDPAFGANSNNPD | NH2 | Genotype C Pre-S1(13-42) |
| 7 | Cmyr-25 | Myr | GTNLSVPNPLGFFPDHQLDPAFGAN | NH2 | Genotype C Pre-S1(13-37) |
| 8 | Cmyr-20 | Myr | GTNLSVPNPLGFFPDHQLDP | NH2 | Genotype C Pre-S1(13-32) |
| 9 | Cmyr-47+(-10) | Myr | GGWSSKPRQGMGTNLSVPNPLGFFPDHQLD PAFGANSNNPDWDFNPNKDHWPEANQVG | NH2 | Genotype C Pre-S1(2-59) |
| 10 | Cmyr-47+(-9) | Myr | GLSWTVPLEWGTNLSVPNPLGFFPDHQLDP AFGANSNNPDWDFNPNKDHWPEANQVG | NH2 | Genotype E or G Pre-S1(2-11) +Genotype C Pre-S1(13-59) |
| 11 | Cplam-47 | Plam | GTNLSVPNPLGFFPDHQLDPAFGANSNNPD WDFNPNKDHWPEANQVG | NH2 | Genotype C Pre-S1(13-59) |
| 12 | Cstea-47 | Stearoyl | GTNLSVPNPLGFFPDHQLDPAFGANSNNPD WDFNPNKDHWPEANQVG | NH2 | Genotype C Pre-S1(13-59) |
| 13 | Cchol-47 | Chol | GTNLSVPNPLGFFPDHQLDPAFGANSNNPD | NH2 | Genotype C Pre-S1(13-59) |
| | | | WDFNPNKDHWPEANQVG | | |
| 14 | Amyr-47 | Myr | GTNLSVPNPLGFFPDHQLDPAFGANSNNPD WDFNPVKDDWPAANQVG | NH2 | Genotype A Pre-S1(13-59) |
| 15 | Bmyr-47 | Myr | GTNLSVPNPLGFFPDHQLDPAFKANSENPD WDLNPNKDNWPDANKVG | NH2 | Genotype B Pre-S1(13-59) |
| 16 | Dmyr-47 | Myr | GQNLSTSNPLGFFPDHQLDPAFRANTANPD WDFNPNKDTWPDANKVG | NH2 | Genotype D Pre-S1(2-48) |
| 17 | Emyr-47 | Myr | GKNISTTNPLGFFPDHQLDPAFRANTRNPD WDHNPNKDHWTEANKVG | NH2 | Genotype E Pre-S1(12-58) |
| 18 | Fmyr-47 | Myr | GQNLSVPNPLGFFPDHQLDPLFRANSSSPD WDFNTNKDSWPMANKVG | NH2 | Genotype F Pre-S1(13-59) |
| 19 | Gmyr-47 | Myr | GKNLSASNPLGFLPDHQLDPAFRANTNNPD WDFNPKKDPWPEANKVG | NH2 | Genotype G Pre-S1(12-58) |
| 20 | Hmyr-47 | Myr | GQNLSVPNPLGFFPDHQLDPLFRANSSSPD WDFNTNKDNWPMANKVG | NH2 | Genotype H Pre-S1(13-59) |
| 51 | Myrcludex B | Myr | GTNLSVPNPLGFFPDHQLDPAFGANSNNPD WDFNPNKDHWPEANKVG | NH2 | Genotype C Pre-S1(13-59) |

It is described that the polypeptide described herein may have at least about 30%, 40%, 50%, 60%, 70%, 80%, 85%, 90%, 95%, 96%, 97%, 98%, or 99% identity to any of the polypeptides described herein. For example, the polypeptide may comprise an amino acid sequence at least about 30%, 40%, 50%, 60%, 70%, 80%, 85%, 90%, 95%, 96%, 97%, 98%, or 99% identical to any one of SEQ ID NOs: 21-40. For example, the polypeptide may comprise an amino acid sequence at least about 30%, 40%, 50%, 60%, 70%, 80%, 85%, 90%, 95%, 96%, 97%, 98%, or 99% identical to any one of SEQ ID NOs: 23, 34-40 and 49. The polypeptide may comprise an amino acid sequence at least about 30%, 40%, 50%, 60%, 70%, 80%, 85%, 90%, 95%, 96%, 97%, 98%, or 99% identical to SEQ ID NO: 23. The variants having certain sequence identity to the polypeptides described herein retain one or more biological activities of the corresponding polypeptides.

Aspects of the present disclosure also include variants of the polypeptides described herein having a native flanking amino acid sequence from the HBV L protein, such as, e.g., from the pre-S1 region of the L protein, added to the N and/or C terminus. The native flanking amino acid sequence refers to the native sequence flanking the N or C terminus of the polypeptide described herein in the pre-S1 region of the corresponding HBV genotype or any other HBV genotypes. In some embodiments, the polypeptide described herein comprises an amino acid sequence chosen from SEQ ID NOs: 23, 34-40 and 49, and a native flanking amino acid sequence at the N and/or C terminus derived from the pre-S1 region of any one of HBV genotypes A-H. In some embodiments, the native flanking amino acid sequence may be derived from the consensus sequence of an HBV strain with the GenBank Accession No. KC875260 (genotype A; SEQ ID NO: 41), AY220704 (genotype B; SEQ ID NO: 42), AF461363 (genotype C; SEQ ID NO: 43), AY796030 (genotype D; SEQ ID NO: 44), AB205129 (genotype E; SEQ ID NO: 45), DQ823095 (genotype F; SEQ ID NO: 46), HE981176 (genotype G; SEQ ID NO: 47), or AB179747 (genotype H; SEQ ID NO: 48). For example, the polypeptide described herein may comprise the amino acid sequence of SEQ ID NO: 23, and a native flanking amino acid sequence at the N and/or C terminus derived from the pre-S1 region of HBV genotype C. Alternatively, the polypeptide described herein may comprise the amino acid sequence of SEQ ID NO: 23, and a native flanking amino acid sequence at the N and/or C terminus derived from the pre-S1 region of any one of HBV genotypes A, B, D, E, F, G, and H. In some embodiments, the N and/or C terminus of the polypeptide described herein may independently comprise a native flanking amino acid sequence having a length of 1-10, such as, e.g., 1-8, 1-5, or 1-3 amino acids, including all integers in between these ranges. For example, the polypeptide described herein may comprise the amino acid sequence of SEQ ID NO: 23 and a native flanking amino acid sequence of 10 amino acids at the N terminus from the pre-S1 region of HBV genotype C. In other words, the polypeptide may comprise amino acids 2-59 of the pre-S1 region of HBV genotype C (SEQ ID NO: 29). As another example, the polypeptide described herein may comprise the amino acid sequence of SEQ ID NO: 23 and a native flanking amino acid sequence of 9 amino acids at the N terminus from the pre-S1 region of HBV genotype E or G. In other words, the polypeptide may comprise amino acids 13-59 of the pre-S1 region of HBV genotype C and amino acids 2-11 of the pre-S1 region of HBV genotype E or G (SEQ ID NO: 30). It will be appreciated that, any polypeptides described herein can have native flanking amino acid sequences of any length extended from the N and/or C terminus, and the resulting polypeptides retain one or more biological activities of the original polypeptides.

The polypeptides described herein can be made by chemical synthesis or by employing recombinant technology.

When recombinant procedures are selected, a synthetic gene may be constructed de novo or a natural gene may be mutated by, for example, cassette mutagenesis. The polypeptides described herein may be produced using recombinant DNA techniques. These techniques contemplate, in simplified form, taking the gene, either natural or synthetic, encoding the peptide; inserting it into an appropriate vector; inserting the vector into an appropriate host cell; culturing the host cell to cause expression of the gene; and recovering or isolating the peptide produced thereby. In some embodiments, the recovered peptide is then purified to a suitable degree.

For example, the DNA sequence encoding a polypeptide described herein is cloned and manipulated so that it may be expressed in a convenient host. DNA encoding parent polypeptides can be obtained from an HBV genomic library, from cDNA derived from mRNA from cells expressing the polypeptide, or by synthetically constructing the DNA sequence. The parent DNA is then inserted into an appropriate plasmid or vector which is used to transform a host cell. In general, plasmid vectors containing replication and control sequences which are derived from species compatible with the host cell are used in connection with those hosts. The vector ordinarily carries a replication site, as well as sequences which encode proteins or peptides that are capable of providing phenotypic selection in transformed cells. The vector may be those commonly used in the art, or constructed using standard techniques by combining functional fragments of the vectors commonly used in the art.

The host cell may be prokaryotic or eukaryotic. For example, prokaryotic host cells may include E. coli, Bacillus subtilis, and other enterobacteriaceae such as, e.g., *Salmonella typhimurium* or *Serratia marcesans,* and various Pseudomonas species. In addition to prokaryotes, eukaryotic organisms, such as yeast cultures, or cells derived from multicellular organisms, such as insect or mammalian cell cultures, may be used. Examples of such eukaryotic host cell lines include VERO and HeLa cells, Chinese Hamster Ovary (CHO) cell lines, W138, 293, BHK, COS-7, and MDCK cell lines.

In some embodiments, the polypeptides described herein may be prepared using solid-phase synthesis, or other equivalent chemical syntheses known in the art. In some embodiments, solid-phase synthesis is initiated from the C-terminus of the peptide by coupling a protected α-amino acid to a suitable resin. Such a starting material can be prepared by attaching an α-amino-protected amino acid by an ester linkage to a chloromethylated resin or a hydroxymethyl resin, or by an amide bond to a BHA resin or MBHA resin. The amino acids are coupled to the peptide chain using techniques well known in the art for the formation of peptide bonds. One method involves converting the amino acid to a derivative that will render the carboxyl group more susceptible to reaction with the free N-terminal amino group of the peptide fragment. For example, the amino acid can be converted to a mixed anhydride by reaction of a protected amino acid with ethylchloroformate, phenyl chloroformate, sec-butyl chloroformate, isobutyl chloroformate, pivaloyl chloride or like acid chlorides. Alternatively, the amino acid can be converted to an active ester such as a 2,4,5-trichlorophenyl ester, a pentachlorophenyl ester, a pentafluorophenyl ester, a p-nitrophenyl ester, a N-hydroxysuccinimide ester, or an ester formed from 1-hydroxybenzotriazole. Another coupling method involves use of a suitable coupling agent such as N,N'-dicyclohexylcarbodiimide or N,N'-diisopropylcarbodiimide.

In some embodiments, the α-amino group of each amino acid employed in the peptide synthesis may be protected during the coupling reaction to prevent side reactions involving their active α-amino function. For example, certain amino acids that contain reactive side-chain functional groups (e.g., sulfhydryl, amino, carboxyl, and hydroxyl) may be protected with suitable protecting groups to prevent a chemical reaction from occurring at that site during both the initial and subsequent coupling steps. The selection of a suitable side-chain protecting group is within the skill of the art. The protecting group will be readily removable upon completion of the desired amino acid peptide under reaction conditions that will not alter the structure of the peptide chain.

After removal of the α-amino protecting group, the remaining α-amino and side-chain protected amino acids are coupled stepwise within the desired order. As an alternative to adding each amino acid separately in the synthesis, some may be coupled to one another prior to addition to the solid-phase synthesizer. The selection of an appropriate coupling reagent is within the skill of the art.

Each protected amino acid or amino acid sequence is introduced into the solid-phase reactor in excess, and the coupling is suitably carried out in a medium of dimethylformamide (DMF) or CH2Cl2 or mixtures thereof. If incomplete coupling occurs, the coupling procedure is repeated before removal of the N-amino protecting group prior to the coupling of the next amino acid. The success of the coupling reaction at each stage of the synthesis may be monitored. The coupling reactions can be performed automatically using well known methods, for example, a BIOSEARCH 9500^{™} peptide synthesizer.

Upon completion of the desired peptide sequence, the protected peptide must be cleaved from the resin support, and all protecting groups must be removed. The cleavage reaction and removal of the protecting groups is suitably accomplished simultaneously or stepwise. When the resin support is a chloro-methylated polystyrene resin, the bond anchoring the peptide to the resin is an ester linkage formed between the free carboxyl group of the C-terminal residue and one of the many chloromethyl groups present on the resin matrix. It will be appreciated that the anchoring bond can be cleaved by reagents that are known to be capable of breaking an ester linkage and of penetrating the resin matrix. It will also be recognized that the polypeptides may be modified (such as, e.g., modified at the N-terminus with a hydrophobic group, including, e.g., myristic acid, palmitic acid, stearic acid, oleic acid, linoleic acid, cholesterol, arachidonic acid; modified at the C-terminus by amidation (amination), isopentanediolization, or other stabilizing C-terminal modification) either before or after the polypeptide is cleaved from the support.

Purification of the polypeptides of the invention may be achieved using conventional procedures such as preparative HPLC (including reversed phase HPLC) or other known chromatographic techniques such as gel permeation, ion exchange, partition chromatography, affinity chromatography (including monoclonal antibody columns) or countercurrent distribution.

### II. Pharmaceutical Compositions and Kits

The present disclosure further provides compositions, including pharmaceutical compositions, comprising a polypeptide described herein. In certain embodiments, the composition may comprise any one or more polypeptides described herein. In some embodiments, the composition may further comprise a suitable pharmaceutically acceptable carrier.

A "pharmaceutically acceptable carrier" refers to an inactive ingredient, such as, e.g., solid, semisolid, or liquid filler, diluent, encapsulating material, formulation auxiliary, excipient, or carrier, for use with a therapeutic agent that together comprise a "pharmaceutical composition" for administration to a subject. A pharmaceutically acceptable carrier is non-toxic to recipients at the dosages and concentrations employed, and is compatible with other ingredients of the formulation. The pharmaceutically acceptable carrier is appropriate for the formulation employed. For example, if the therapeutic agent is to be administered orally, the carrier may be a gel capsule. If the therapeutic agent is to be administered subcutaneously, the carrier ideally is not irritable to the skin and does not cause injection site reaction.

Pharmaceutical compositions of the polypeptides described herein may be prepared by mixing such polypeptide having the desired degree of purity with one or more optional pharmaceutically acceptable carriers. Pharmaceutically acceptable carriers may include, e.g.: buffers (such as, e.g., phosphate, citrate, and other organic acids); antioxidants (such as, e.g., ascorbic acid and methionine); preservatives (such as, e.g., octadecyldimethylbenzyl ammonium chloride, hexamethonium chloride, benzalkonium chloride, benzethonium chloride, phenol, butyl or benzyl alcohol, alkyl parabens such as methyl or propyl paraben, catechol, resorcinol, cyclohexanol, 3-pentanol, and m- cresol); low molecular weight (such as, e.g., less than about 10 residues) polypeptides; proteins (such as, e.g., serum albumin, gelatin, or immunoglobulins); hydrophilic polymers (such as, e.g., polyvinylpyrrolidone); amino acids (such as, e.g., glycine, glutamine, asparagine, histidine, arginine, or lysine); monosaccharides, disaccharides, and other carbohydrates including glucose, mannose, or dextrins; chelating agents (such as, e.g., EDTA); sugars (such as, e.g., sucrose, mannitol, trehalose or sorbitol); salt-forming counter-ions (such as, e.g., sodium); metal complexes (such as, e.g., Zn- protein complexes); and/or non-ionic surfactants (such as, e.g., polyethylene glycol (PEG)).

Exemplary pharmaceutical carriers may also include binding agents (such as, e.g., pregelatinized maize starch, polyvinylpyrrolidone or hydroxypropyl methylcellulose, etc.); fillers (such as, e.g., lactose and other sugars, microcrystalline cellulose, pectin, gelatin, calcium sulfate, ethyl cellulose, polyacrylates or calcium hydrogen phosphate, etc.); lubricants (such as, e.g., magnesium stearate, talc, silica, colloidal silicon dioxide, stearic acid, metallic stearates, hydrogenated vegetable oils, corn starch, polyethylene glycols, sodium benzoate, sodium acetate, etc.); disintegrants (such as, e.g., starch, sodium starch glycolate, etc.); and wetting agents (such as, e.g., sodium lauryl sulphate, etc.).

Exemplary pharmaceutically acceptable carriers may further include interstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.). In some embodiments, a sHASEGP may be combined in the pharmaceutical composition with one or more additional glycosammoglycanases, such as, e.g., chondroitinases.

The pharmaceutical compositions may also comprise more than one active ingredient suitable for the particular indication being treated, for example, those with complementary activities that do not adversely affect each other. Such active ingredients may be suitably present in combination in amounts that are effective for the purpose intended.

In some embodiments, the active ingredients may be entrapped in microcapsules prepared, for example, by coacervation techniques or by interfacial polymerization, such as, e.g., hydroxymethylcellulose or gelatin-microcapsules and poly-(methylmethacylate) microcapsules, respectively, in colloidal drug delivery systems (such as e.g., liposomes, albumin microspheres, microemulsions, nano-particles and nanocapsules), or in macroemulsions.

In some embodiments, the pharmaceutical composition may comprise sustained-release preparations. Suitable examples of sustained-release preparations include, e.g., semipermeable matrices of solid hydrophobic polymers containing the polypeptides described herein, which matrices may be in the form of shaped articles, such as, e.g., films or microcapsules.

In some embodiments, the pharmaceutical compositions may be used for *in vivo* administration and may be sterile. Sterility may be readily accomplished, e.g., by filtration through sterile filtration membranes.

The pharmaceutical compositions may be formulated into any of many possible dosage forms, such as, e.g., tablets, capsules, gel capsules, powders, or granules. The pharmaceutical compositions may also be formulated as solutions, suspensions, emulsions, or mixed media. In some embodiments, the pharmaceutical compositions may be formulated as lyophilized formulations or aqueous solutions.

In some embodiments, the pharmaceutical compositions may be formulated as a solution. For example, the polypeptides described herein may be administered in an unbuffered solution, such as, e.g., in saline or in water. In some embodiments, the polypeptides may also be administered in a suitable buffer solution. For example, the buffer solution may comprise acetate, citrate, prolamine, carbonate, or phosphate, or any combination thereof. In some embodiments, the buffer solution may be phosphate buffered saline (PBS). The pH and osmolality of the buffer solution containing the polypeptides can be adjusted to be suitable for administering to a subject.

In some embodiments, the pharmaceutical compositions may be formulated as suspensions in aqueous, non-aqueous, or mixed media. Aqueous suspensions may further contain **substances** which increase the viscosity of the suspension including, for example, sodium carboxymethylcellulose, sorbitol and/or dextran. The suspension may also contain stabilizers.

In some embodiments, the pharmaceutical compositions may be formulated as emulsions. Exemplary emulsions include heterogeneous systems of one liquid dispersed in another in the form of droplets usually exceeding 0.1 µm in diameter. Emulsions may contain additional components in addition to the dispersed phases, and the active drug which may be present in a solution in the aqueous phase, the oily phase, or itself as a separate phase. Microemulsions are also included as an embodiment of the present disclosure. In some embodiments, the pharmaceutical compositions may also be formulated as liposomal formulations.

In some embodiments, the pharmaceutical compositions may be formulated as a dosage form suitable for administration, e.g., an injection suitable for subcutaneous administration. In these embodiments, the pharmaceutical compositions comprise a certain amount of the polypeptide described herein, so as to achieve a therapeutically effective amount of a daily dosage after the administration of one or several doses of the pharmaceutical compositions per day. A therapeutically effective amount herein refers to an amount of the polypeptide at which the saturating content of the polypeptide in liver target organ can be achieved after several days (≥ 7 days) of administration of the therapeutically effective amount of the polypeptide.

In some embodiments, the therapeutically effective amount described herein is a daily dosage of 777.95-926.13 nmol. The therapeutically effective amount described herein is a daily dosage of 4.2~5.0 mg.

In some embodiments, the polypeptide in the pharmaceutical composition is hepalatide, and the therapeutically effective amount is 4.2~5.0 mg per day, preferably 4.2 mg per day. In some embodiments, the polypeptide in the pharmaceutical composition is Myrcludex B, and the therapeutically effective amount is 4.2~5.0 mg per day, preferably 5.0 mg per day.

In some embodiments, the pharmaceutical compositions described herein are administrated several times per day, and therapeutically effective amount can be achieved. Thus, in these embodiments, the pharmaceutical compositions described herein are prepared as multiple unit dosage forms, e.g., each unit dosage form may comprise at least 18.52 nmol, or may comprise 37.05 nmol, 55.57 nmol, 111.14 nmol, 129.66 nmol, 259.32 nmol or 388.98 nmol. Corresponding number of the unit dosage forms may be administrated per day, to achieve a total dosage of the day in the range of the therapeutically effective amount. In these embodiments, when the pharmaceutical compositions comprise hepalatide, each unit dosage form may comprise at least 0.1 mg of hepalatide, or may comprise 0.2 mg, 0.3 mg, 0.6 mg, 0.7 mg, 1.4 mg, 2.1 mg or 4.2 mg of hepalatide, i.e., the specification of the pharmaceutical compositions is 0.1 mg, 0.2 mg, 0.3 mg, 0.6 mg, 0.7 mg, 1.4 mg, 2.1 mg or 4.2 mg. For example, when each unit dosage form comprises 0.1 mg of hepalatide, 42 unit dosage forms of the pharmaceutical compositions may be administrated per day. In these embodiments, when the pharmaceutical compositions comprise Myrcludex B, each unit dosage form may comprise at least 0.1 mg of Myrcludex B, or may comprise 0.25 mg, 0.5 mg, 1.0 mg, 2.5 mg or 5.0 mg of Myrcludex B, i.e., the specification of the pharmaceutical compositions is 0.1 mg, 0.25 mg, 0.5mg, 1.0 mg, 2.5 mg or 5.0 mg. For example, when each unit dosage form comprises 0.1 mg of Myrcludex B, 50 unit dosage forms of the pharmaceutical compositions may be administrated per day. Thus, in the pharmaceutical compositions of the present invention, the number of the unit dosage forms may be in the range of 1~50 doses, such as 1~42 doses.

In some embodiments, each unit dosage form may comprise 777.95-926.13 nmol or 4.2~5.0 mg of the polypeptide described herein. Thus, the therapeutically effective amount may be achieved by administrating 1 unit dosage form per day.

The pharmaceutical compositions described herein may be in a form of lyophilized preparation. For example, a dose of lyophilized medicaments may comprise 37.05~926.13 nmol or 0.1~5 mg of the polypeptide described herein, e.g., 259.32~926.13 nmol or 1.4~5 mg, 388.98~926.13 nmol or 2.1~5 mg, or 777.95-926.13 nmol or 4.2~5 mg of the polypeptide described herein. When used, it can be administrated as an injection to a subject after dissolving in an appropriate amount of solvent. One or several doses of the injection described herein may be administrated, to obtain a daily total dosage in the range of 777.95-926.13 nmol or 4.2~5 mg. For example, a lyophilized preparation may comprise two bottles of medicaments, each bottle comprises 388.98 mmol or 2.1 mg of lyophilized polypeptides, and the preparation may be administered to the subject twice a day, to obtain a daily total dosage of 777.95 nmol or 4.2 mg.

Further described is a kit comprising the pharmaceutical compositions. One or several doses of the pharmaceutical compositions described herein may be comprised in the kit for preventing or treating hepatitis B virus-related liver diseases by one or more days of administration.

It is described that he kit comprises at least 7 doses of the pharmaceutical compositions, and each dose of the pharmaceutical compositions comprises 777.95-926.13 nmol or 4.2~5.0 mg of the polypeptide described herein, for continuous administration for at least 7 days. For example, the kit enables 7 days of continuous administration.

For example, the kit comprises at least 7 doses of the pharmaceutical compositions, and each dose of the pharmaceutical compositions comprises 4.2~5.0 mg, preferably 4.2 mg of hepalatide or 5.0 mg of Myrcludex B, for continuous administration for at least 7 days.

For example, the kit comprises at least 2 doses of the pharmaceutical compositions, and the total amount of the polypeptide comprised in the pharmaceutical compositions in the kit reaches 777.95-926.13 nmol or 4.2~5.0 mg, as the total dosage per dayIt is described that each dose of the pharmaceutical compositions may comprise e.g. 18.52 nmol, 37.05 nmol, 55.57 nmol, 111.14 nmol, 129.66 nmol, 259.32 nmol or 388.98 nmol, or 0.1 mg, 0.2 mg, 0.3 mg, 0.6 mg, 0.7 mg, 1.4 mg or 2.1 mg of the polypeptide, and the kit may comprise at least 42 doses, 21 doses, 14 doses, 7 doses, 6 doses, 3 doses or 2 doses of the pharmaceutical compositions; alternatively, each dose of the pharmaceutical compositions may comprise 18.52 nmol, 46.31 nmol, 92.61 nmol, 185.23 nmol or 463.06 nmol, or 0.1 mg, 0.25 mg, 0.5 mg, 1.0 mg or 2.5 mg of the polypeptide, and the kit may comprise at least reach 50 doses, 20 doses, 10 doses, 5 doses or 2 doses of the pharmaceutical compositions.

For example, the kit comprises 2 or more doses of the pharmaceutical compositions, the polypeptide in the pharmaceutical compositions is hepalatide, the amount of hepalatide in each dose of the pharmaceutical compositions is 0.1 mg, 0.2 mg, 0.3 mg, 0.6 mg, 0.7 mg, 1.4 mg or 2.1 mg, and the total amount of the polypeptide comprised in the pharmaceutical compositions in the kit is 4.2 mg.

For example, the kit comprises 2 or more doses of the pharmaceutical compositions, the polypeptide in the pharmaceutical compositions is hepalatide, the amount of Myrcludex B in each dose of the pharmaceutical compositions is 0.1 mg, 0.2 mg, 0.3 mg, 0.6 mg, 0.7 mg, 1.4 mg or 2.1 mg, and the total amount of the polypeptide comprised in the pharmaceutical compositions in the kit is 5.0 mg.

It is described that the kit comprises multiple doses of the pharmaceutical compositions, which enables at least 7 days of continuous administration, and a total amount of the polypeptide comprised in several doses of the multiple doses of the pharmaceutical compositions meets the daily dosage, i.e., reaches 777.95-926.13 nmol or 4.2~5.0 mg. For example, in some embodiments, each dose of the pharmaceutical compositions comprises 388.98 nmol or 2.1 mg of the polypeptide, and the kit comprises at least 14 doses of the pharmaceutical compositions, and every 2 doses meet the daily dose. When each dose of the pharmaceutical compositions comprises 259.32 nmol or 1.4 mg of the polypeptide, the kit comprises at least 21 doses of the pharmaceutical compositions, and every 3 doses meet the daily dose, and so on. For example, the polypeptide comprised by the pharmaceutical compositions is hepalatide or Myrcludex B. For example, the medicaments described herein enable 7 days of continuous administration.

The pharmaceutical compositions of the invention and the kit described herein are used for administrating to a subject in need thereof the polypeptide described herein, so that the content of the polypeptide in his/her liver target organs is saturated after at least 7 days of continuous administration.

### III. Methods of Use

Embodiments of the present disclosure include therapeutic and prophylactic uses of the polypeptides described herein. In one aspect, use of the polypeptides described herein as a medicament is provided. In another aspect, use of the polypeptides described herein in treating and preventing HBV-related liver diseases is provided.

The hepatitis B virus-related diseases describe herein include chronic hepatitis B virus infection, chronic hepatitis B, hepatitis B-related liver fibrosis and cirrhosis, hepatitis B-related liver cancer, hepatitis B-related liver transplantation and hepatitis B-related mother-to-child transmission. The chronic hepatitis B described herein includes HBeAg positive chronic hepatitis B and HBeAg negative chronic hepatitis B. In some embodiments, the hepatitis B-related liver transplantation described herein includes the protection of the donor liver from HBV infection before, during and after transplantation.

"Patient" and "subject" may be used interchangeably to refer to an animal, such as a mammal or a human, being treated or assessed for a disease, disorder, or condition, at risk of developing a disease, disorder, or condition, or having or suffering from a disease, disorder, or condition. In some embodiments, such disease, disorder, or condition may include metabolic diseases. In some embodiments, the metabolic diseases may involve a lipid metabolism disorder.

A sufficient dosage of the polypeptides or the pharmaceutical compositions thereof described herein may be administrated to a subject, for preventing or treating HBV-related liver diseases. The "sufficient dosage" described herein refers to a daily dosage of a polypeptide at which the content of the polypeptide in liver target organ is saturated after continuous administration of such daily dosage of the polypeptide for several days (≥ 7 days). In these embodiments, the "sufficient dosage" described herein is usually a daily dosage of 777.95-926.13 nmol or 4.2~5.0 mg, such as 777.95-833.52 nmol or 4.2~4.5 mg, or 777.95 nmol or 4.2 mg, or 926.13 nmol or 5.0 mg. The content of the polypeptide in the target organ is not saturated when such dosage of the polypeptide is first administrated, but is saturated after 7 continuous administrations. Thus, the preventing or treating methods described herein comprise a step of continuous administrating the sufficient dosage of the polypeptides or the pharmaceutical compositions comprising the polypeptide for at least 7 days.

"Target organ saturation" or "... in the target organ is saturated" described herein refers to after administrating a certain amount of the polypeptides, an apparent volume of distribution is less than 100 L. In these embodiments, the apparent volume of distribution is usually 10~100 L, such as 10~50 L or 20~40 L.

In various embodiments, the term "treatment" includes treatment of a subject (e.g. a mammal, such as a human) or a cell to alter the current course of the subject or cell. Treatment includes, e.g., administration of a polypeptide described herein or a pharmaceutical composition comprising such polypeptide, and may be performed either prophylactically or subsequent to the initiation of a pathologic event or contact with an etiologic agent. Also included are "prophylactic" treatments, which can be directed to reducing the rate of progression of the disease or condition being treated, delaying the onset of that disease or condition, or reducing the severity of its onset. "Treatment" or "prophylaxis" does not necessarily indicate complete eradication, cure, or prevention of the disease or condition or the associated symptoms. In various embodiments, the term "treatment" may include relieving, alleviating, or reversing the pathological processes of a HBV-related liver disease. In some embodiments, the term "treatment" may include improving at least one symptom or measurable parameter of a metabolic disease. It will be apparent to one of skill in the art which biological and/or physiological parameters can be used to access the pathological process of metabolic disease. Such pathological processes or symptoms may include, e.g., excessive or increased levels compared with healthy subjects of one or more chemical or biological molecules associated with metabolism, such as, e.g., ALT, HBV DNA; or of one or more physiological parameters that measure metabolic changes, such as, e.g., liver function index.

The terms "administering," or "administer" include delivery of the polypeptide described herein to a subject either by local or systemic administration. Administration may be topical (including ophthalmic and to mucous membranes including vaginal and rectal delivery), pulmonary (e.g., by inhalation or insufflation of powders or aerosols, including by nebulizer, intratracheal, intranasal), epidermal, transdermal, oral, or parenteral. Parenteral administration includes intravenous, subcutaneous, intraperitoneal, or intramuscular injection or infusion; or intracranial, e.g., intrathecal or intraventricular, administration.

The present disclosure also provides methods to carry out the above uses of the polypeptides described herein in a subject. Such methods may comprise administering to the subject a therapeutically or prophylactically effective amount of a polypeptide described herein or of a pharmaceutical composition comprising such polypeptide. In some embodiments, the subject may be a mammal. In some embodiments, the subject may be a human. In some embodiments, the subject may suffer from a HBV-related liver disease or may be at risk of developing such disease.

Such combination therapies described herein may encompass combined administration (where two or more therapeutic agents are included in the same or separate formulations), and separate administration, in which case, administration of the polypeptides described herein can occur prior to, simultaneously, and/or following, administration of the additional therapeutic agent. Such medicaments include interferon, nucleotide medicaments, hepatitis B immunoglobulin, and hepatitis B vaccine. Interferon includes common interferon and PEG long-acting interferon. Common interferon includes interferon α-2a and interferon α-2b. PEG long-acting interferon includes PEG interferon α-2a and PEG interferon α-2b. Nucleotide medicaments include lamivudine LAM, entecavir ETV, tenofovir disoproxil TDF, telbivudine TBV, adefovir dipivoxil ADV, and tenofovir alafenamide TAF.

The polypeptides described herein (and any additional therapeutic agent) can be administered by any suitable means, including parenteral, intrapulmonary, and intranasal, and, if desired for local treatment or intralesional administration. In some embodiments, the polypeptides described herein may be parenterally administered. Parenteral administration may include intramuscular, intravenous, intraarterial, intraperitoneal, or subcutaneous administration. In some embodiments, the polypeptides described herein may be administered subcutaneously. In some embodiments, the polypeptides described herein may be administered intravenously. Dosing can be by any suitable route, such as, e.g., by injections or infusions, such as intravenous or subcutaneous injections or infusions, depending in part on whether the administration is brief or chronic. Various dosing schedules including e.g. single or multiple administrations over various time-points, bolus administration, and pulse infusion are also contemplated.

Therefore, the present disclosure also includes the polypeptides and the pharmaceutical compositions thereof described herein which is used in the methods described herein for treating or preventing hepatitis B-related liver diseases. More specifically, the polypeptides or the pharmaceutical compositions thereof described herein is used in the therapeutic and prophylactic methods described herein at a daily dosage of 777.95-926.13 nmol or 4.2~5.0 mg (calculated by polypeptide), and is continuously administrated for several days, preferably for at least 7 days, to achieve liver target organ saturation.

In some embodiments, the present disclosure includes the hepalatide and the pharmaceutical compositions thereof described herein which is used in the methods described herein for treating or preventing hepatitis B-related liver diseases, wherein the methods described herein includes administrating hepalatide to a subject at a daily dosage of 777.95-926.13 nmol or 4.2~5.0 mg, preferably 777.95 nmol or 4.2 mg, preferably administrating Myrcludex B to a subject at a daily dosage of 926.13 nmol or 5.0 mg, and continuously administrating for several days, preferably for at least 7 days, to achieve liver target organ saturation.

Preferably, the pharmaceutical compositions are pharmaceutical compositions described in any embodiment of the present disclosure.

The following Examples may be used for illustrative purposes and should not be deemed to narrow the scope of the invention.

### Example 1: Synthesis of Polypeptides

Polypeptides can be synthesized according to the method of Example 1.1 of PCT/CN2017/086558. Specifically, polypeptides as shown in Table 1 were synthesized according to the standard Fmoc protocol for polypeptide synthesis. Generally, individual amino acid residues were extended from the carboxyl terminus to the amino terminus, starting from a MBHA resin. The N terminus was then modified by myristoylation. After completion of peptide synthesis, the polypeptides were cleaved from the resin by a cleavage solution and the C terminus of the polypeptides was further modified by amination. The resin was removed by filtering with G6 sand-core funnel and the filtrate containing the polypeptides was dried under vacuum. The polypeptide product was dissolved in deionized water, and purified in ÄKTA explorer 100 type medium pressure liquid chromatography equipped with C18 column. The main peaks were recovered stepwise. The samples collected from the target peak were analyzed by Agilent 1100 type reversed phase high pressure liquid chromatography (HPLC) equipped with C18 column for their purities and confirmed by mass spectrometry for their molecular weights. The collected solutions purified by medium pressure liquid chromatography were freeze-dried for storage. The dried samples were dissolved in PBS and then filtered through a 0.20 µM membrane. The polypeptide stocks dissolved in PBS were stored at -80 °C before use. The amino acid sequence of hepalatide is shown in SEQ ID NO: 23, its N terminus is myristoylated, its C terminus is aminated, the molecular weight is 5398.8 Da, and the molecular formula is C₂₄₇H₃₅₂N₆₅O₇₃.

### Example 2: Blocking HBV Infection in vitro by Polypeptides

Free tupaia primary hepatocytes were obtained from anesthetized adult male tupaias by microsurgical technique using hepatic portal vein collagenase two-step perfusion method, and cultured using a dedicated hepatocyte culture system. Hepatocytes cultured for 3 days were added with purified HBV virus to infect hepatocytes with multiple MOI = 0, 6.25, 12.5, 25, 50 and 100 genomic DNA/cell. It can be seen that the secretion amount of HBeAg in the supernatant after 12 days has a well linear relationship with MOI (R² = 0.9873), confirming that HBsAg in the supernatant can quantitatively reflect the degree of HBV infection.

Hepalatide (0, 2.5, 5, 10, 20, 40 ng/ml) was added to block HBV infection simultaneously when hepatocytes is infected by HBV (MOI = 100 genomic DNA/cell). It can be seen that the secretion amount of HBeAg in the supernatant decreases with the increase of the concentration of hepalatide, and logit-log regression analysis shows that the two have a well linear correlation dose-dependent relationship (R² = 0.9563). Hepalatide inhibits HBV infection in a dose-dependent manner. At this time, the calculated median inhibitory concentration IC50 of hepalatide is 3.956 ng/ml.

### Example 3: Blocking HBV Infection in Model Animals by Polypeptides

50 adult male tupaias were randomly divided into 5 groups: PBS control group, hepalatide high (2 mg/kg), medium (0.4 mg/kg) and low (0.08 mg/kg) dose group and hepatitis B immunoglobulin HBIG block group (60 IU/kg). The tupaias were infected by intraperitoneal injection of HBV virus serum at a titer of 108 HBV DNA/ml. The hepalatide block groups were given subcutaneous injections of different dosages of hepalatide on days 0, 1, 2, 3, 5, 7, 9, 11, and 13 after infection. The HBIG block group was given intramuscular injection of immunoglobulin HBIG on the day of infection and the third day after infection. Serums of the tupaias were collected 4 days before infection and on days 9, 14, 21, and 42 after infection, and HBsAg, HBeAg, HBV DNA titer and alanine aminotransferase (ALT) in the serums were detected. Liver tissue was taken for pathological examination on the 21st day after infection.

HBsAg peaked on the 9th day after infection in the PBS control group, and was basically cleared on the 15th day. Hepalatide (high, medium and low dose group) can effectively block HBV infection, so that HBsAg was well controlled during infection. HBIG cannot effectively block HBV infection of the infectious dosage. On the 9th day after infection, HBsAg in the HBIG block group did not decrease significantly as compared with the PBS control group, and there was a tendency to prolong HBsAg secretion. On the 15th day, the HBsAg level in the HBIG block group was higher than that in the PBS control group. The HBsAg levels in the hepalatide (high, medium and low dose) groups on the 9th day were all lower than that in the PBS control group and that in the HBIG block group, wherein the HBsAg levels in the hepalatide (high, medium and low dose) groups on the 9th day were all significantly lower than that in the HBIG block group (P < 0.05).

HBeAg peaked on the 9th day after infection in the PBS control group, and disappeared basically on the 21th day. Hepalatide can effectively block HBV infection, and HBsAg was well controlled by high-dose and medium-dose hepalatide during infection. HBIG cannot effectively block HBV infection of the infectious dosage. HBsAg in the HBIG block group on the 9th day and 15th day was equivalent to that in the PBS control group. The HBeAg levels in the hepalatide (high, medium and low dose) groups on the 9th day were all lower than that in the PBS control group and that in the HBIG block group, wherein the HBsAg levels in the hepalatide (high, medium and low dose) groups on the 9th day were all significantly lower than that in the PBS control group (P < 0.05).

HBV DNA peaked on the 9th day after infection in the PBS control group, and was cleared on the 15th day. Hepalatide can effectively block HBV infection, and HBV DNA was well controlled by high-dose and medium-dose hepalatide during infection. HBIG cannot effectively block HBV infection of the infectious dosage. HBV DNA in the HBIG block group was further increased as compared to the PBS control group on the 15th day after infection, and did not tend to clear until 21 days after infection. The HBV DNA levels in the hepalatide (high, medium and low dose) groups on the 9th day were all lower than that in the PBS control group and that in the HBIG block group, wherein the HBV DNA level in the hepalatide high dose group was significantly lower than that in the HBIG block group (P < 0.05).

The mean value of ALT before infection was 56.8 IU/ml, and this value was considered as the normal ALT level of tupaia. In the PBS control group, ALT peaked on the 21th day after infection, and returned to normal level on the 42th day. Hepalatide can effectively block HBV infection, and ALT was well controlled by high-dose and medium-dose hepalatide during infection. HBIG cannot effectively block HBV infection of the infectious dosage. ALT in the HBIG block group was still higher than the normal level on the 21th day after infection, and returned to normal until 42 days after infection. The ALT levels in the hepalatide (high, medium and low dose) groups on the 21th day were all lower than that in the PBS control group and that in the HBIG block group, wherein the ALT levels in the hepalatide high and medium dose groups were significantly lower than that in the HBIG block group (P < 0.05).

One tupaia of each experimental group was randomly selected for liver histopathological examination on the 21th day after infection, and liver tissue of healthy tupaia was used as normal control. The PBS group showed significant pathological changes of tupaia liver tissue caused by HBV infection, mainly including turbid and swollen hepatocytes and balloon-like changes at the portal area around hepatic lobule, with a small amount of lymphocyte infiltration, which are very similar to hepatitis caused by HBV acute infection in adults. HBIG cannot alleviate the pathological changes of tupaia liver tissue caused by HBV infection. The pathological changes of liver in the low dose hepalatide group were similar to that in the PBS group, with the degree of lesions slightly alleviated. High-dose and medium-dose hepalatides completely blocked the pathological damage to hepatocytes caused by HBV like balloon-like changes, etc, and the liver tissues were basically the same as the liver tissue of healthy tupaia.

In conclusion, hepalatide can effectively block HBV infection in the terms of serology (HBsAg, HBeAg), virology (serum HBV DNA copy number), enzymology (alanine aminotransferase, ALT) and pathology.

### Example 4: Specific Binding of Polypeptides to NTCP

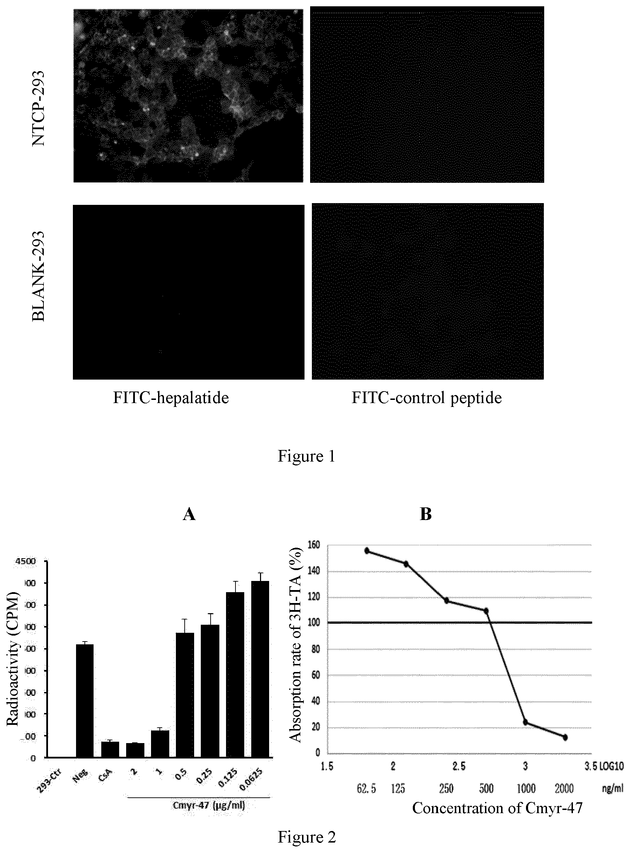

NTCP-293 cell line was formed by transfecting non-hepatocyte-derived 293 cells with NTCP. Amino acid K at position 37 of hepalatide (Cmyr-47) or unrelated control peptide (peptide 47 derived from the Pre-S1 region of heron HBV, myristoyl-GLNQSTFNPLGFFPSHQLDPLFKANAGSADWDKNPNKDPWP QAHDTA-amido, SEQ ID NO: 50) were fluorescently labeled by FITC. As shown in Fig. 1, FITC-hepalatide bound to NTCP-293 cells, but did not bind to 293 cells which was not transfected with NTCP (BLANK-293), illustrating that the binding of hepalatide to cells is NTCP specific. FITC-labeled unrelated control peptide did not bind to NTCP-293 cells, illustrating that the binding is specific to the sequence of hepalatide.

### Example 5: Effect of Polypeptides on Bile Acid Transport Function of NTCP

Effect of hepalatide on the bile acid transport function of NTCP was studied according to the assessment of bile acid transport function of NTCP (Kim RB et al. J Pharmacol Exp Ther. 1999 Dec; 291(3): 1204-9). Using 293 cells transfected with human NTCP (NTCP-293) as the cell model, the effect of hepalatide on NTCP-293 uptake of ³H-labeled bile acid (³H-TA) was studied. Results show that (see Fig. 2A): 1) TA entry into cells is specifically mediated by NTCP, because 293 cells themselves have little absorption of TA; 2) the positive control cyclosporin A exerted a significant inhibitory effect on NTCP, consistent with a reported result; 3) hepalatide showed a bidirectional effect on NTCP (see Fig. 2B) that at low concentration (< 500ng/ml, equivalent to 50×IC50) hepalatide promotes the absorption of TA, while at high concentration (> 500ng/ml) hepalatide inhibit the absorption of TA, and the IC50 concentration is 838.81 ng/ml.

### Example 6: Preclinical Pharmacokinetic Study of Polypeptides

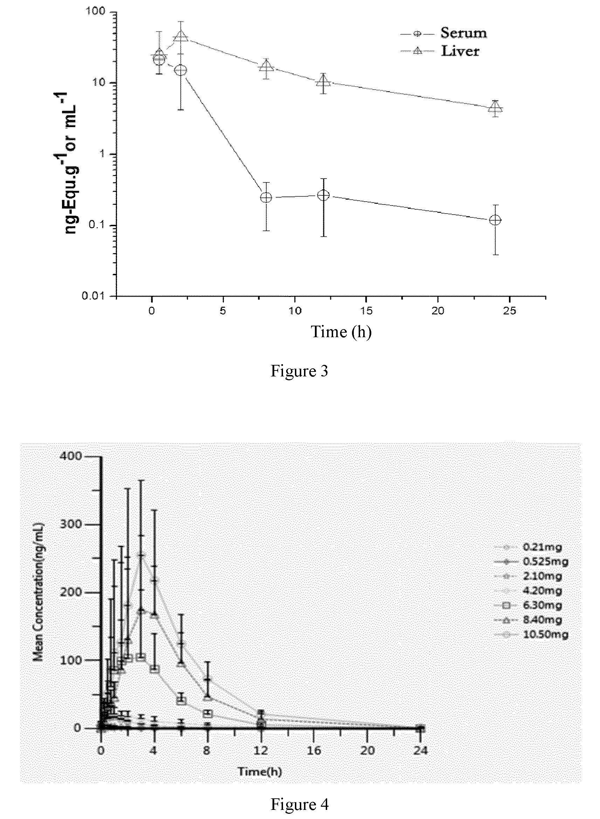

After ¹²⁵I-labeled hepalatide was administrated to rats by a single subcutaneous injection at a dosage of 40 ug·kg⁻¹, the distribution of hepalatide in various tissues was detected. Results (Fig. 3) show that hepalatide was mainly distributed in the liver and urinary systems, wherein hepalatide was mainly in the form of metabolites in urinary system. The medicament reached its peak within 2 hours in the liver, and Cmax is 44.3±28.9ng-Equ.mL⁻¹. Its terminal elimination half-life t1/2 in the liver is as long as 8.7 h, and a higher concentration in the liver at 24 h (4.5±1.2 ng-Equ.mL⁻¹) was observed. The medicament concentration in the liver at 48 h is estimated to be about 0.66 ng-Equ.mL⁻¹, which is equivalent to the serum medicament concentration 4 hours after subcutaneous injection. The medicament in the serum was cleared quickly, and substantially decreased to the baseline 6 hours after administration. The results of preclinical pharmacokinetics show that hepalatide is significantly accumulated in liver target organ.

### Example 7: Single-dose Climb Phase Ia Clinical Trial and Clinical Pharmacokinetic Study of Polypeptide

Administration/exposure: The single-dose climb phase Ia clinical trial of hepalatide was a randomized, double-blind, blank-controlled, single-center, dose-climbing trial. Six dose groups (0.525 mg, 2.1 mg, 4.2 mg, 6.3 mg, 8.4 mg and 10.5 mg) were set up, and 5, 5, 10, 10, 10 and 5 healthy subjects were planned to be enrolled in each group. The ratio of men to women is 22:23. After screening, the healthy subjects were enrolled into the experimental groups and the control group double-blindly and randomly at the ratio of 4: 1, and were administrated by a single subcutaneous injection of hepalatide, and were observed for 7 days after drug withdrawal. The administrations and exposures of hepalatide during the test were shown in Table 2.

Number and characteristics of subjects: 45 adult healthy subjects were planned to be enrolled in the groups, and 45 subjects were actually enrolled. All of them completed the trial and were included in the safety analysis. The population for safety analysis of phase Ia clinical trial was shown in Table 3. There were no significant differences in demographic indicators such as age, gender, ethnicity, occupation, height, weight, BMI and past history among the groups.

**Table 2. Overall design and dosage settings of phase Ia trial**

| Phase | Subject | Dosage (s.c) | Number Test drug : placebo (sex ratio) |
|---|---|---|---|
| Ia | Healthy subjects | 0.525 mg/person | 4:1 (male 2, female 3) |
| | | 2.10 mg/person | 4:1 (male 3, female 2) |
| | | 4.20 mg/person | 8:2 (Male and female half and half) |
| | | 6.30 mg/person | 8:2 (Male and female half and half) |
| | | 8.40 mg/person | 8:2 (Male and female half and half) |
| | | 10.50 mg/person | 4:1 (male 2, female 3) |

**Table 3. Demographic indicators of single-dose climb phase Ia clinical trial**

| | | Double-blind phase | | | | | | |
|---|---|---|---|---|---|---|---|---|
| Dosage | | Blank control | 0.525 mg | 2.1 mg | 4.2 mg | 6.3 mg | 8.4 mg | 10.5 mg |
| Subject enrolled N | | **9** | **4** | **4** | **8** | **8** | **8** | **4** |
| Sex | Male | 5 | 1 | 3 | 4 | 3 | 4 | 2 |
| | Female | 4 | 3 | 1 | 4 | 5 | 4 | 2 |
| nationality | Han nationality | 7 | 4 | 3 | 8 | 8 | 8 | 4 |
| | Non-Han nationality | 2 | 1 | 1 | 0 | 0 | 0 | 0 |
| Profession | Physical | 0 | 0 | 0 | 0 | 0 | 0 | 0 |
| | Non-physical | 9 | 4 | 4 | 8 | 8 | 8 | 4 |
| Age (year) | Mean | 27.56 | 28.75 | 25.50 | 27.38 | 25.50 | 25.63 | 22.50 |
| Height (cm) | Mean | 162.17 | 160.50 | 165.88 | 164.38 | 163.19 | 162.56 | 164.00 |
| Weight (kg) | Mean | 60.49 | 56.08 | 59.45 | 63.75 | 57.81 | 60.30 | 59.80 |
| BMI | Mean | 23.02 | 21.78 | 21.48 | 23.63 | 21.69 | 22.93 | 22.23 |
| Past-history | No | 9 | 4 | 4 | 7 | 7 | 8 | 4 |
| | Yes | 0 | 0 | 0 | 1 | 1 | 0 | 0 |

Safety research: No deaths or serious adverse events occurred in the single-dose climb phase Ia clinical trial of hepalatide. A total of 63 adverse events were recorded during the trial, all of which were grade I adverse events. There are 29 cases of adverse events that were related to, likely related and possibly related to the test drug (see Table 4). The incidence of adverse events was not significantly different between each dose group and the blank control group. The incidence of adverse events in the 8.4 mg dose group was significantly higher than that in the blank control group. There was no significant difference in the incidence of adverse reactions between the 0.525 mg, 2.10 mg, 4.20 mg, 6.30 mg, 10.50 mg dose groups and the blank control group.

**Table 4. Distribution of phase Ia adverse events (certainly related, likely related, and possibly related) in each test group**

| Adverse event n (%) | Total | Group | | | | | | |
|---|---|---|---|---|---|---|---|---|
| | | Blank control | 0525mg | 2.1mg | 4.2mg | 6.3mg | 8.4mg | 10.5mg |
| Subject | 45 | 9 | 4 | 4 | 8 | 8 | 8 | 4 |
| Total bile acid increase | 14(31.1) | 0(0.0) | 0(0.0) | 0(0.0) | 3(37.5) | 2(25.0) | 6(75.0) | 3(75.0) |
| Blood phosphorous increase | 3(6.7) | 0(0.0) | 0(0.0) | 0(0.0) | 1(12.5) | 0(0.0) | 2(25.0) | 0(0.0) |
| Urobilinogen increase | 2(4.4) | 0(0.0) | 0(0.0) | 0(0.0) | 0(0.0) | 0(0.0) | 0(0.0) | 2(50.0) |
| Urinary bilirubin increase | 2(4.4) | 0(0.0) | 0(0.0) | 0(0.0) | 0(0.0) | 0(0.0) | 0(0.0) | 2(50.0) |
| White blood cell count decrease | 2(4.4) | 1(11.1) | 0(0.0) | 0(0.0) | 0(0.0) | 1(12.5) | 0(0.0) | 0(0.0) |
| Neutrophil decrease | 2(4.4) | 1(11.1) | 0(0.0) | 0(0.0) | 0(0.0) | 1(12.5) | 0(0.0) | 0(0.0) |
| Blood fibrinogen decrease | 1(2.2) | 0(0.0) | 0(0.0) | 0(0.0) | 0(0.0) | 1(12.5) | 0(0.0) | 0(0.0) |
| Blood prolactin increase | 1(2.2) | 0(0.0) | 0(0.0) | 0(0.0) | 0(0.0) | 1(12.5) | 0(0.0) | 0(0.0) |
| Hemoglobin decrease | 1(2.2) | 0(0.0) | 0(0.0) | 0(0.0) | 0(0.0) | 1(12.5) | 0(0.0) | 0(0.0) |
| Blood uric acid increase | 1(2.2) | 0(0.0) | 0(0.0) | 0(0.0) | 1(12.5) | 0(0.0) | 0(0.0) | 0(0.0) |
| Total (including TBA increase) | 29(64.4) | 2(22.2) | 0(0.0) | 0(0.0) | 5(62.5) | 7(87.5) | 8(100.0) | 7(175.0) |

Phase Ia clinic pharmacokinetic study: A phase Ia clinical pharmacokinetic study of single-dose climbing subcutaneous administration was carried out in 45 healthy subjects. The dosage settings and PK analysis population are shown in Table 5. Blood samples were collected before and 2 minutes, 5 minutes, 15 minutes, 30 minutes, 45 minutes, 1 hour, 1.5 hours, 2 hours, 3 hours, 4 hours, 6 hours, 8 hours, 12 hours, and 24 hours after administration. The medicament concentrations in the biological samples were detected by liquid-mass spectrometry with the limit of quantitation of 0.2 ng/ml. The blood medicament concentration curve is shown in Fig. 4, and the main parameters of blood pharmacokinetics are shown in Table 4. Study results show that after the subject received a single subcutaneous injection of hepalatide, the blood medicament curve was low and plat after receiving a single subcutaneous injection of hepalatide in the dosage range of 0.525 mg-4.2 mg, while the blood medicament curve is substantially raised in the dosage range of 6.3 mg-10.5 mg. In the dosage range of 0.21-4.2 mg, the apparent distribution volume Vz/F is greater than 100 L, suggesting that the medicament was mainly distributed in the target organs. Combined with the preclinical study showing the specific binding of hepalatide to NTCP (Example 4), and the preclinical pharmacokinetic study showing that hepalatide is significantly aggregated in liver(Example 6), it can be seen that the drug target NTCP is expressed specifically in liver, so it is determined that the medicaments were mainly aggregated in the target organ liver. In the dose range of 6.3-10.5 mg, Vz/F is close to the total body fluid volume of the human body, and shows a decreasing trend with increasing dosage, indicating that the medicament is distributed to the body fluid after the medicament is saturated in the target organ. Therefore, single administration dosages of 0.525 mg-4.2 mg and 6.3 mg-10.5 mg are unsaturated dosage range and saturated dosage range of target organ liver, respectively. An apparent distribution volume of 135 L was shown after a single subcutaneous injection of 5 mg Myrcludex B (lank A et al. J Hepatol. 2016 Sep; 65 (3): 483-9). According to the results of this example, Myrcludex B administered subcutaneously at such dose did not reach liver target organ saturation.

**Table 5. Main pharmacokinetic parameters of phase Ia PK study**

| Parameter (unit) | Dosage | | | | | |
|---|---|---|---|---|---|---|
| | 0.525 mg | 2.10 mg | 4.20 mg | 6.30 mg | 8.40 mg | 10.5 mg |
| | (n=4) | (n=4) | (n=8) | (n=8) | (n=8) | (n=4) |
| Tₘₐₓ (h) | 0.38 | 0.88 | 1.00 | 3.50 | 3.50 | 3.00 |
| Cₘₐₓ (ng/mL) | 4.2135 | 16.8513 | 23.2838 | 123.8361 | 193.4533 | 259.4850 |
| kₑₗ (l/h) | 0.2769 | 0.2729 | 0.1918 | 0.2656 | 0.2406 | 0.2505 |
| t_{1/2} (h) | 2.61 | 2.65 | 3.98 | 2.68 | 2.90 | 2.84 |
| AUC₀₋ₜ(h*ng/mL) | 9.9877 | 52.9378 | 118.4435 | 581.8290 | 1002.396 | 1422.484 |
| AUC_{0-inf} (h*ng/mL) | 11.2527 | 55.9433 | 121.9178 | 585.0795 | 1007.109 | 1428.830 |
| %AUCₑₓ (h*ng/mL) | 11.50 | 5.17 | 3.31 | 0.77 | 0.48 | 0.53 |
| V_{z}/F (L) | 179.7523 | 142.3606 | 227.7019 | 53.9407 | 42.3828 | 34.8535 |
| CL/F (L/h) | 47.0963 | 38.4002 | 39.0537 | 13.5085 | 10.1147 | 8.1971 |
| Cₘₐₓ/dose (ng/mL/mg) | 8.0257 | 8.0244 | 5.5438 | 19.6565 | 23.0301 | 24.7129 |
| AUC_{0-inf}/dose (h*ng/mL/mg) | 21.4336 | 26.6396 | 29.0280 | 92.8698 | 119.8940 | 136.079 |

### Example 8: Multiple-dose Climb Phase Ib Clinical Trial and Clinical Pharmacokinetic Study of Polypeptide

Administration/exposure: The multiple-dose climb phase Ib clinical trial of hepalatide was a randomized, double-blind, blank-controlled, single-center trial. Three dose groups (4.20 mg, 6.30 mg and 8.40 mg) were set up, and 10, 10 and 15 healthy subjects were planned to be enrolled in each group, respectively. The ratio of men to women is 18:17. After screening, the healthy subjects were enrolled into the experimental groups and the blank control group double-blindly and randomly at the ratio of 4: 1, and were administrated by subcutaneous injection once a day for 7 days, and were observed for 3 days after drug withdrawal. The administrations and exposures of hepalatide during the test were shown in Table 6.

Number and characteristics of subjects: 35 adult healthy subjects were planned to be enrolled in the groups in the phase Ib clinical trial of hepalatide, and 35 subjects were actually enrolled. All of them completed the trial and were included in the safety analysis. The population for safety analysis of phase Ib clinical trial and demographic indicators of each test group were shown in Table 7. There were no significant differences in demographic indicators such as age, gender, ethnicity, occupation, height, weight, BMI and past history among the groups.

**Table 6. Overall design and dosage settings of phase Ib trial**

| Phase | Subject | Dosage (s.c) | Number Test drug : placebo (sex ratio) |
|---|---|---|---|
| Ib | Healthy subjects | 4.20 mg/person | 8:2 (Male and female half and half) |
| | | 6.30 mg/person | 8:2 (Male and female half and half) |
| | | 8.40 mg/person | 12:3 (male 8, female 7) |

**Table 7. Demographic basic informations of phase Ib clinical trial**

| Variate | | Group | | | |
|---|---|---|---|---|---|
| | | Blank control | 4.2mg | 6.3mg | 8.4mg |
| Number n (miss) | | 7(0) | 8(0) | 8(0) | 12(0) |
| Sex | Male | 4 | 4 | 4 | 6 |
| | Female | 3 | 4 | 4 | 6 |
| Nationality | Han Nationality | 7 | 8 | 8 | 11 |
| | Non-Han Nationality | 0 | 0 | 0 | 1 |
| Profession | Physical | 0 | 0 | 0 | 1 |
| | Non-physical | 7 | 8 | 8 | 11 |
| Age (year) | Mean | 30.29(6.07) | 27.88(4.36) | 29.38(8.21) | 30.42(8.58) |
| Height (cm) | Mean | 162.71 | 162.38 | 160.31 | 160.83 |
| Weight (kg) | Mean | 60.16 | 60.69 | 59.49 | 58.58 |
| BMI | Mean | 22.77(2.31) | 22.89(2.84) | 23.20(2.23) | 22.53(2.45) |
| Past-history | No | 7 | 8 | 8 | 12 |
| | Yes | 0 | 0 | 0 | 0 |
| Allergy history | No | 7 | 8 | 8 | 12 |
| | Yes | 0 | 0 | 0 | 0 |

Safety evaluation: A total of 74 adverse events were recorded during the phase Ib clinical trial of hepalatide, all of which were grade I adverse events, not treated and relieved by themselves. A total of 36 adverse events that were related to, likely related and possibly related to the test medicament were recorded (see Table 8). The main adverse event was total bile acid increase, and a total of 27 such events accounted for 75.0% of all adverse events. Since the increase of bile acid is a pharmacological response of hepalatide, after deducting the adverse events of total bile acid increase, the incidence of adverse events in the 4.2, 6.3, and 8.4 mg dose groups was 25.0%, 37.5%, and 8.3%, respectively, equivalent to the incidence of adverse events in the blank control group of 42.9%. No dose-limiting toxicity DLT of hepalatide occurred during the phase Ib clinical study, and the maximum tolerated dose MTD was not reached when the dosage climbed to the maximum dosage of 8.4 mg in this study.

**Table 8. Distribution of phase Ib adverse events (certainly related, likely related, and possibly related) in each test group**

| Adverse event n(%) | Group | | | | |
|---|---|---|---|---|---|
| | Total | Blank control | 4.2 mg | 6.3 mg | 8.4 mg |
| Subject n | 35 | 7 | 8 | 8 | 12 |
| Total bile acid increase | 27(77.1) | 0(0.0) | 8(100.0) | 8(100.0) | 11(91.7) |
| Blood phosphorous increase | 6(20.0) | 2(28.6) | 1(12.5) | 2(25.0) | 1(8.3) |
| Blood fibrinogen decrease | 1(2.9) | 0(0.0) | 1(12.5) | 0(0.0) | 0(0.0) |
| Blood creatine phosphokinase increase | 1(2.9) | 1(14.3) | 0(0.0) | 0(0.0) | 0(0.0) |
| Conjugated bilirubin increase | 1(2.9) | 0(0.0) | 0(0.0) | 1(12.5) | 0(0.0) |
| Total | 36(102.9) | 3(42.9) | 10(125.0) | 11(112.5) | 12(100.0) |

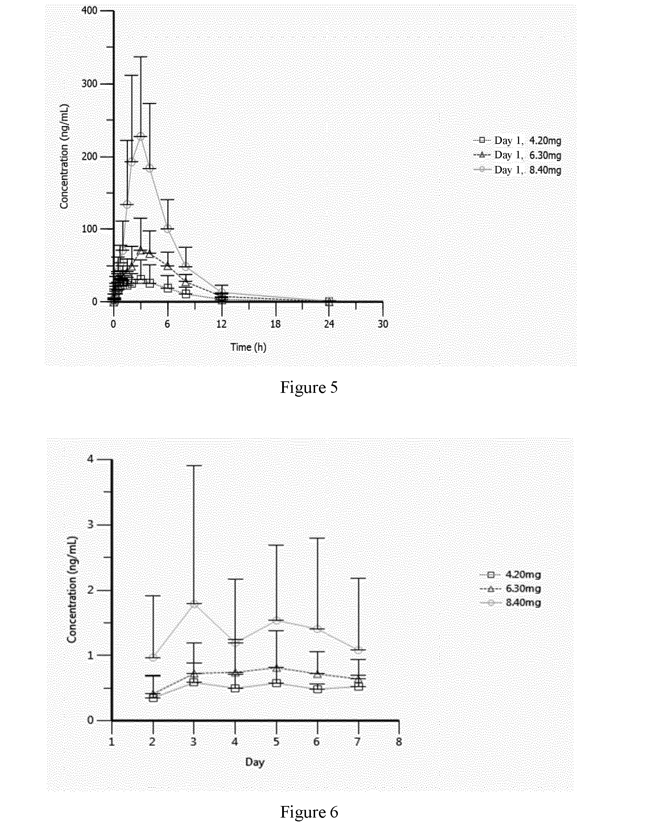

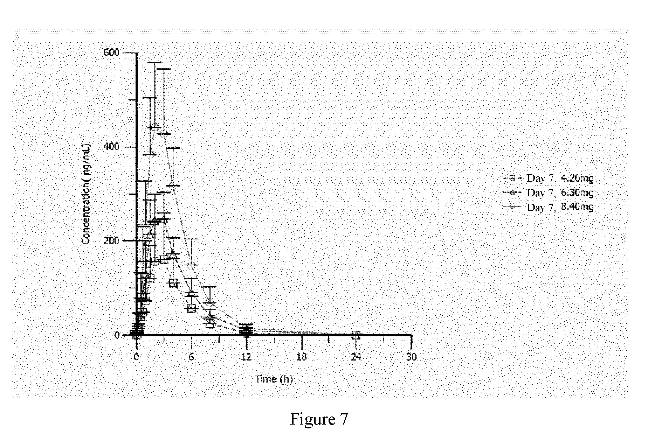

Clinic pharmacokinetic study: A phase Ib clinical pharmacokinetic study of multiple-dose climbing subcutaneous administration was carried out in 35 healthy subjects. Subjects were administered continuously once a day for 7 days. The dosage settings and PK analysis population are shown in Table 9. Blood samples were collected at the following times: before and 2 minutes, 5 minutes, 15 minutes, 30 minutes, 45 minutes, 1 hour, 1.5 hours, 2 hours, 3 hours, 4 hours, 6 hours, 8 hours, and 12 hours after administration; Day 2: 24 hours after Day 1 administration (before Day 2 administration of); Days 3~6: before administration; Day 7: before and 2 minutes, 5 minutes, 15 minutes, 30 minutes, 45 minutes, 1 hour, 1.5 hours, 2 hours, 3 hours, 4 hours, 6 hours, 8 hours, and 12 hours after administration. The medicament concentrations in the biological samples were detected by liquid-mass spectrometry with the limit of quantitation of 0.2 ng/ml. The blood sample medicament-time curves after the first doses, before the Days 2-7 administrations and after the last doses are shown in Figs. 5-7, and the main pharmacokinetic parameters are shown in Table 10. Results show that after the first doses, the blood medicament curve of each dose group was consistent with that of phase Ia, and the first dose of 4.2 mg did not achieve target organ saturation, and the first dose of 6.3 and 8.4 mg achieved target organ saturation. The medicament valley concentration reached a steady state during the continuous administration on days 2-6. After 7 days of continuous administration, the accumulation coefficients R_{AUC} of the 4.2 mg, 6.3 mg, and 8.4 mg dose groups were 4.43 ± 1.60, 2.76 ± 0.76, and 1.91 ± 0.39, respectively, indicating that hepalatide exhibited an accumulation effect during continuous administration. After accumulation, the apparent distribution volume in the 4.2 mg dose group decreased from 153.6025 ± 107.1807 liters at the first dose to 22.1880 ± 8.0721 liters at the last dose, indicating that the last dose at a dosage of 4.2 mg achieved target organ saturation after continuous administration and accumulation. After continuous administration at a maximum dose of 8.4 mg and fully accumulation in the target organ, the apparent volume of distribution was 15.1021 ± 4.4743 liters, which was close to the 14 liters of extravascular fluid in the body, indicating that the medicaments outside the target organ were mainly distributed in the extracellular fluid.

**Table 9. PK population of phase Ib clinical pharmacokinetic analysis**

| PK population | Group | | | |
|---|---|---|---|---|
| | Blank control | 4.2mg | 6.3mg | 8.4mg |
| Male n (%) | 4(57.14%) | 4(50.00%) | 4(50.00%) | 6(50.00%) |
| Female n (%) | 3(42.86%) | 4(50.00%) | 4(50.00%) | 6(50.00%) |
| Total n (%) | 7(100.00%) | 8(100.00%) | 8(100.00%) | 12(100.00%) |

**Table 10. Main pharmacokinetic parameters of phase Ib PK study**

| Parameter (unit) | Day | Dosage | | |
|---|---|---|---|---|
| | | 4.20 mg | 6.30 mg | 8.40 mg |
| Tₘₐₓ(h) | Day 1 | 1.50 (0.50-6.00) | 3.00 (0.50-6.00) | 3.00 (2.00-6.00) |
| | Day 7 | 2.50 (2.00-3.00) | 2.00 (1.50-3.00) | 2.00 (2.00-3.00) |
| Cₘₐₓ (ng/mL) | Day 1 | 36.5183±24.1333 | 77.3095±39.8130 | 243.4407±99.8722 |
| | Day 7 | 177.2036±103.3422 | 261.2495±56.8376 | 471.7638±145.2946 |
| t_{1/2} (h) | Day 1 | 3.38±0.63 | 2.5 6±0.32 | 2.64±0.48 |
| | Day 7 | 2.45±0.54 | 2.53±0.33 | 2.46±0.43 |
| AUC₀₋₂₄ (h*ng/mL) | Day 1 | 211.8645±147.9841 | 475.4362±177.0202 | 1162.3100±485.5221 |
| | Day 7 | 750.0411±331.7298 | 1217.2184±233.6315 | 2103.8784±573.5105 |
| AUC₀₋₂₄/dose (h*ng/mL/mg) | Day 1 | 50.4439±35.2343 | 75.4661±28.0985 | 138.3702±57.8002 |
| | Day 7 | 178.5812±78.9833 | 193.20934+37.0844 | 250.4617±68.2751 |
| V_{z}/F (L) | Day 1 | 153.6025±107.1807 | 54.5848±20.6336 | 30.8332±10.6378 |
| | Day 7 | 22.1880±8.0721 | 19.3358±3.6093 | 15.1021±4.4743 |
| CL/F (L/h) | Day 1 | 30.1377±18.4428 | 14.8595±5.3876 | 8.1221±2.5669 |
| | Day 7 | 6.3941±2.1992 | 5.3304±0.9344 | 4.2628±1.1101 |
| R_{Cmax} | Day 1 | 5.34±1.55 | 3.95±1.6 | 2.07±0.48 |
| R_{AUC} | Day 7 | 4.43±1.60 | 2.76±0.76 | 1.91±0.39 |

Clinic pharmacokinetic study: In the multiple-dose climb phase Ib clinical trial, the subjects received continuous subcutaneous injections of hepalatide once a day for 7 days at the dosage range of 4.2-8.4 mg, and during the administration period, the levels of total blood bile acids were effectively increased and returned to normal after drug withdrawal. The total bile acid can be used as an ideal biomarker. Study results show that a sufficient dosage to achieve pharmacodynamic response can be reached by continuous subcutaneous injection of 4.2 mg of hepalatide per day for 7 days.

### Example 9: Summary of Clinical Pharmacokinetics of Polypeptide Phase I Clinical Trial

Hepalatide is mainly distributed in the target organ after a single subcutaneous injection in the dosage range of 0.525-4.2 mg; after a single subcutaneous injection in the dosage range of 6.3-10.5 mg, the target organ is saturated and the drug is distributed to plasma. Surprisingly, when hepalatide was administered at a dosage of 4.20 mg in a single subcutaneous injection, the target organ saturation in the liver was not achieved, but after continuously administering by subcutaneous injection for 7 days at such dosage, the liver target organ was saturated after accumulation, which is never reported and unpredictable. According to the results disclosed in the present application, a daily dosage of 4.2 mg was determined as the lowest target organ saturation dosage for continuous administration of hepalatide. Based on the results of the present disclosure, liver target organ saturation was not achieved by a single subcutaneous administration at a dosage of 5 mg of Myrcludex B, but through continuous administration at such dosage, liver target organ saturation would be achieved after accumulation.

### Example 10: Summary of Safety of Peptide Phase I Clinical Trail

The subjects were well tolerated with a single subcutaneous injection of hepalatide in the dosage range of 0.525-10.5 mg, no dose-limiting toxicity occurred, and the maximum tolerated dosage was not reached. The subjects were well tolerated with continuous subcutaneous injections of hepalatide for 7 days at the dosage range of 4.2-8.4 mg per day, no dose-limiting toxicity occurred, and the maximum tolerated dosage was not reached.

### Example 11: Clinically recommended dosage

Since hepalatide can reach saturating level in liver target organ after continuous administration at dosage of 4.2 mg per day and accumulation, hepalatide at such dosage is clinically safe, and the pharmacodynamic biomarker response of total bile acid is also achieved, such dosage is demonstrated to be the clinically recommended dosage.

## Claims

1. A polypeptide comprising an amino acid sequence selected from SEQ ID NOs: 21 - 40 and 49 or a pharmaceutical composition comprising said polypeptide for use in a method for treating or preventing HBV related liver diseases, wherein the polypeptide is administered in a dose of 4.2 - 5.0mg for at least 7 consecutive days.

2. The polypeptide or a pharmaceutical composition comprising the polypeptide for use of claim 1, wherein the polypeptide comprises an N-terminal modification with a hydrophobic group; preferably, the hydrophobic group is chosen from myristic acid, palmitic acid, stearic acid, oleic acid, linoleic acid, cholesterol, and arachidonic acid; more preferably, the hydrophobic group is myristic acid; and/ or the polypeptide comprises a C-terminal modification; preferably, the C-terminal modification is amidation, isopentanediolization or no-modification.

3. The polypeptide or a pharmaceutical composition comprising the polypeptide for use of claim 1 or 2, wherein the polypeptide comprises at the N-terminus and/or the C-terminus a native flanking amino acid sequence from the pre-S1 region of HBV; preferably, the native flanking amino acid sequence from the pre-S1 region of HBV has 1-10, 1-8, 1-5, or 1-3 amino acids in length.

4. The polypeptide or a pharmaceutical composition comprising the polypeptide for use of claim 3, wherein the polypeptide comprises an N-terminal modification with a hydrophobic group chosen from myristic acid, palmitic acid, stearic acid, and cholesterol, and a C-terminal modification with amidation; preferably, the polypeptide is set forth in any one of SEQ ID NOs: 1-20 or 51; more preferably, the polypeptide is set forth in SEQ ID NO: 3 or 51.

5. The polypeptide or a pharmaceutical composition comprising the polypeptide for use of any one of claims 1-4, wherein the polypeptide is administered in a daily dosage of 4.2 - 5.0 mg, preferably 4.2 mg or 5.0 mg, for at least 7 consecutive days.

6. The polypeptide or a pharmaceutical composition comprising the polypeptide for use of claim 5, wherein
the polypeptide comprises the amino acid sequence of SEQ ID NO: 23, and comprises an N-terminal modification with myristic acid and a C-terminal modification with amination; preferably the sufficient dosage is a daily dosage of 4.2 mg; or
the polypeptide comprises the amino acid sequence of SEQ ID NO: 49, and comprises an N-terminal modification with myristic acid and a C-terminal modification with amination, preferably the sufficient dose is a daily dosage of 5.0 mg.

7. The polypeptide or a pharmaceutical composition comprising the polypeptide for use of claim 1, wherein
the polypeptide comprises the amino acid sequence of SEQ ID NO: 23, and comprises an N-terminal modification with myristic acid and a C-terminal modification with amination, the method comprises administering N doses of the pharmaceutical composition to the subject per day to achieve a daily dosage of 4.2 mg, wherein N is any integer from 1 to 42; or
the polypeptide comprises the amino acid sequence of SEQ ID NO: 49, and comprises an N-terminal modification with myristic acid and a C-terminal modification with amination, the method comprises administering N doses of the pharmaceutical composition to the subject per day to achieve a daily dosage of 5.0 mg, wherein N is any integer from 1 to 50.

8. The polypeptide or a pharmaceutical composition comprising the polypeptide for use of any one of claims 1-7, wherein the HBV related liver diseases include chronic hepatitis B virus infection, chronic hepatitis B, hepatitis B-related liver fibrosis and cirrhosis, hepatitis B-related liver cancer; preferably, the chronic hepatitis B includes HBeAg positive chronic hepatitis B and HBeAg negative chronic hepatitis B.

## Patentansprüche

1. Polypeptid, umfassend eine Aminosäuresequenz, ausgewählt aus SEQ ID Nr: 21 - 40 und 49, oder eine pharmazeutische Zusammensetzung, umfassend das Polypeptid, zur Verwendung in einem Verfahren zur Behandlung oder Vorbeugung von HBV-bedingten Lebererkrankungen, wobei das Polypeptid in einer Dosis von 4,2 - 5,0 mg für mindestens 7 aufeinanderfolgende Tage verabreicht wird.

2. Polypeptid oder pharmazeutische Zusammensetzung, umfassend das Polypeptid zur Verwendung nach Anspruch 1, wobei das Polypeptid eine N-terminale Modifikation mit einer hydrophoben Gruppe umfasst; wobei vorzugsweise die hydrophobe Gruppe ausgewählt ist aus Myristinsäure, Palmitinsäure, Stearinsäure, Ölsäure, Linolsäure, Cholesterin und Arachidonsäure; wobei bevorzugter die hydrophobe Gruppe Myristinsäure ist; und/oder das Polypeptid eine C-terminale Modifikation umfasst; wobei vorzugsweise die C-terminale Modifikation Amidierung, Isopentandiolierung oder keine Modifikation ist.

3. Polypeptid oder pharmazeutische Zusammensetzung, umfassend das Polypeptid zur Verwendung nach Anspruch 1 oder 2, wobei das Polypeptid am N-Terminus und/oder am C-Terminus eine native flankierende Aminosäuresequenz aus der pre-S1-Region von HBV umfasst; wobei vorzugsweise die native flankierende Aminosäuresequenz aus der pre-S1-Region von HBV eine Länge von 1-10, 1-8, 1-5 oder 1-3 Aminosäuren hat.

4. Polypeptid oder pharmazeutische Zusammensetzung, umfassend das Polypeptid zur Verwendung nach Anspruch 3, wobei das Polypeptid eine N-terminale Modifikation mit einer hydrophoben Gruppe, ausgewählt aus Myristinsäure, Palmitinsäure, Stearinsäure und Cholesterin, und eine C-terminale Modifikation mit Amidierung umfasst; wobei vorzugsweise das Polypeptid in einer der SEQ ID Nr: 1-20 oder 51 dargelegt ist; wobei bevorzugter das Polypeptid in SEQ ID Nr: 3 oder 51 dargelegt ist.

5. Polypeptid oder pharmazeutische Zusammensetzung, umfassend das Polypeptid zur Verwendung nach einem der Ansprüche 1 bis 4, wobei das Polypeptid in einer täglichen Dosierung von 4,2 bis 5,0 mg, vorzugsweise 4,2 mg oder 5,0 mg, für mindestens 7 aufeinanderfolgende Tage verabreicht wird.

6. Polypeptid oder pharmazeutische Zusammensetzung, umfassend das Polypeptid zur Verwendung nach Anspruch 5, wobei
das Polypeptid die Aminosäuresequenz von SEQ ID Nr: 23 umfasst und eine N-terminale Modifikation mit Myristinsäure und eine C-terminale Modifikation mit Aminierung umfasst; wobei vorzugsweise die ausreichende Dosierung eine tägliche Dosierung von 4,2 mg ist; oder
das Polypeptid die Aminosäuresequenz von SEQ ID Nr: 49 umfasst und eine N-terminale Modifikation mit Myristinsäure und eine C-terminale Modifikation mit Aminierung umfasst, wobei vorzugsweise die ausreichende Dosis eine tägliche Dosis von 5,0 mg ist.

7. Polypeptid oder pharmazeutische Zusammensetzung, umfassend das Polypeptid zur Verwendung nach Anspruch 1, wobei
das Polypeptid die Aminosäuresequenz von SEQ ID Nr: 23 umfasst und eine N-terminale Modifikation mit Myristinsäure und eine C-terminale Modifikation mit Aminierung umfasst, wobei das Verfahren das Verabreichen von N Dosen der pharmazeutischen Zusammensetzung an den Patienten pro Tag umfasst, um eine Tagesdosis von 4,2 mg zu erreichen, wobei N eine ganze Zahl von 1 bis 42 ist; oder
das Polypeptid die Aminosäuresequenz von SEQ ID Nr: 49 umfasst und eine N-terminale Modifikation mit Myristinsäure und eine C-terminale Modifikation mit Aminierung umfasst, wobei das Verfahren das Verabreichen von N Dosen der pharmazeutischen Zusammensetzung an den Patienten pro Tag umfasst, um eine Tagesdosis von 5,0 mg zu erreichen, wobei N eine ganze Zahl von 1 bis 50 ist.

8. Polypeptid oder pharmazeutische Zusammensetzung, umfassend das Polypeptid zur Verwendung nach einem der Ansprüche 1 bis 7, wobei die HBV-bedingten Lebererkrankungen chronische Hepatitis-B-Virusinfektion, chronische Hepatitis B, Hepatitis-B-bedingte Leberfibrose und -zirrhose, Hepatitis-B-bedingten Leberkrebs umfassen; wobei vorzugsweise die chronische Hepatitis B HBeAg-positive chronische Hepatitis B und HBeAgnegative chronische Hepatitis B einschließt.

## Revendications

1. Polypeptide comprenant une séquence d'acides aminés choisie parmi les SEQ ID NO : 21 à 40 et 49 ou composition pharmaceutique comprenant ledit polypeptide, pour une utilisation dans une méthode de traitement ou de prévention de maladies hépatiques associées au VHB, dans lequel le polypeptide est administré à une dose de 4,2 à 5,0 mg pendant au moins 7 jours consécutifs.

2. Polypeptide ou composition pharmaceutique comprenant le polypeptide pour l'utilisation selon la revendication 1, dans lequel le polypeptide comprend une modification N-terminale avec un groupe hydrophobe ; de préférence le groupe hydrophobe est choisi parmi l'acide myristique, l'acide palmitique, l'acide stéarique, l'acide oléique, l'acide linoléique, le cholestérol, et l'acide arachidonique ; mieux encore le groupe hydrophobe est l'acide myristique ; et/ou le polypeptide comprend une modification C-terminale ; de préférence la modification C-terminale est une amidation, une isopentanediolisation ou une absence de modification.

3. Polypeptide ou composition pharmaceutique comprenant le polypeptide pour l'utilisation selon la revendication 1 ou 2, dans lequel le polypeptide comprend, à l'extrémité N et/ou à l'extrémité C, une séquence d'acides aminés flanquante native issue de la région pré-S1 du VHB ; de préférence la séquence d'acides aminés flanquante native issue de la région pré-S1 du VHB a une longueur de 1 à 10, 1 à 8, 1 à 5 ou 1 à 3 acides aminés.

4. Polypeptide ou composition pharmaceutique comprenant le polypeptide pour l'utilisation selon la revendication 3, dans lequel le polypeptide comprend une modification N-terminale avec un groupe hydrophobe choisi parmi l'acide myristique, l'acide palmitique, l'acide stéarique, et le cholestérol, et une modification C-terminale avec une amidation ; de préférence le polypeptide est présenté dans l'une quelconque des SEQ ID NO : 1 à 20 et 51 ; mieux encore le polypeptide est présenté dans la SEQ ID NO : 3 ou 51.

5. Polypeptide ou composition pharmaceutique comprenant le polypeptide pour l'utilisation selon l'une quelconque des revendications 1 à 4, dans lequel le polypeptide est administré à une dose quotidienne de 4,2 à 5,0 mg, de préférence de 4,2 mg ou 5,0 mg, pendant au moins 7 jours consécutifs.

6. Polypeptide ou composition pharmaceutique comprenant le polypeptide pour l'utilisation selon la revendication 5, dans lequel
le polypeptide comprend la séquence d'acides aminés de la SEQ ID NO : 23, et comprend une modification N-terminale avec de l'acide myristique et une modification C-terminale avec une amination ; de préférence la dose suffisante est une dose quotidienne de 4,2 mg ; ou
le polypeptide comprend la séquence d'acides aminés de la SEQ ID NO : 49, et comprend une modification N-terminale avec de l'acide myristique et une modification C-terminale avec une amination, de préférence la dose suffisante est une dose quotidienne de 5,0 mg.

7. Polypeptide ou composition pharmaceutique comprenant le polypeptide pour l'utilisation selon la revendication 1, dans lequel
le polypeptide comprend la séquence d'acides aminés de la SEQ ID NO : 23, et comprend une modification N-terminale avec de l'acide myristique et une modification C-terminale avec une amination, la méthode comprenant l'administration de N doses de la composition pharmaceutique au sujet chaque jour pour que soit atteinte une dose quotidienne de 4,2 mg, où N est un entier quelconque de 1 à 42 ; ou
le polypeptide comprend la séquence d'acides aminés de la SEQ ID NO : 49, et comprend une modification N-terminale avec de l'acide myristique et une modification C-terminale avec une amination, la méthode comprenant l'administration de N doses de la composition pharmaceutique au sujet chaque jour pour que soit atteinte une dose quotidienne de 5,0 mg, où N est un entier quelconque de 1 à 50.

8. Polypeptide ou composition pharmaceutique comprenant le polypeptide pour l'utilisation selon l'une quelconque des revendications 1 à 7, dans lequel les maladies hépatiques associées au VHB comprennent une infection chronique par le virus de l'hépatite B, une hépatite B chronique, une cirrhose et une fibrose hépatique associées à une hépatite B, un cancer hépatique associé à une hépatite B ; de préférence l'hépatite B chronique comprend une hépatite B chronique positive à HBeAg et une hépatite B chronique négative à HBeAg.
